# EUROPEAN PATENT APPLICATION

(11) **EP 3 973 965 A1**
(43) Date of publication of application: **30.03.2022**
(21) Application number: 20819259.1
(22) Date of filing: 29.05.2020
(51) Int. Cl.: A61K 31/704, A61K 31/7048, A61K 31/7024, A61P 1/16, A61P 1/00, A61P 29/00, A61P 11/00, A61P 3/10, A61P 13/12, A61P 1/04, A61P 11/06, A61P 11/02, A61P 37/08, A61P 9/00, A61P 3/00, A61P 3/06, A61P 3/04, A61P 9/10, A61P 27/02, A61P 25/00, A61P 19/06

(54) **MEDICAL USE OF PENTACYCLIC TRITERPENOID SAPONIN COMPOUND AND PHARMACEUTICAL COMPOSITION THEREOF**

(30) Priority: 06.06.2019 CN 201910495350
(71) Applicant: China Pharmaceutical University, Nanjing, Jiangsu 210009 (CN)
(72) Inventor: SUN, Hongbin, Nanjing, Jiangsu 210009 (CN); LIU, Liu, Nanjing, Jiangsu 210009 (CN); LI, Haobin, Nanjing, Jiangsu 210009 (CN); DAI, Liang, Nanjing, Jiangsu 210009 (CN); HU, Kaiwen, Nanjing, Jiangsu 210009 (CN); LIU, Jun, Nanjing, Jiangsu 210009 (CN); LIN, Chao, Nanjing, Jiangsu 210009 (CN); WEN, Xiaoan, Nanjing, Jiangsu 210009 (CN)
(74) Representative: Sun, Yiming
(86) International application number: PCT/CN2020/093230
(87) International publication number: WO 2020/244454

(57) **Abstract**

The present invention discloses a medical use and a drug composition for pentacyclic triterpenoid saponin compounds, and particularly relates to a use of pentacyclic triterpenoid saponins represented by formulas (I)-(XVI) in preparing a drug for preventing or treating AMPK and/or ERRα-mediated diseases, including in preparing a drug for preventing or treating diseases, such as hepatic diseases, respiratory system diseases, metabolic disorders, autoimmune diseases, cardiovascular and cerebrovascular diseases, kidney diseases, central nervous system diseases or muscular dystrophy. The drug composition for preventing and treating the AMPK and/or ERRa-mediated diseases in the present invention contains compounds represented by formulas (I)-(XVI) or pharmaceutically acceptable salts or solvates thereof as active ingredients and pharmaceutically acceptable adjuvants.

## Description

### Technical Field

The present invention belongs to the scope of medicines and particularly relates to a use of natural pentacyclic triterpenoid saponin in preparing a drug for preventing or treating AMPK and/or ERRa-mediated diseases and a drug composition. These diseases include hepatic diseases, respiratory system diseases, metabolic disorders, autoimmune diseases, cardiovascular and cerebrovascular diseases, kidney diseases, central nervous system diseases or muscular dystrophy.

### Background Art

Hepatic diseases, respiratory system diseases, metabolic disorders, autoimmune diseases, cardiovascular and cerebrovascular diseases, kidney diseases, central nervous system diseases and muscular dystrophy are very complex in pathological mechanism but all have a common pathological mechanism, i.e. histiocyte death (including apoptosis, necroptosis, pyroptosis and ferroptosis), inflammatory responses and fibrosis caused by various acute or chronic stress stimulation, and occurrence and development of these diseases are both closely related to abnormal regulation of AMPK and/or ERRα signaling pathways.

AMPK (Adenosine Monophosphate-Activated Protein Kinase) is a key kinase for regulating energy metabolism and stress responses of an organism, phosphorylation activation of the AMPK may overcome insulin resistance, blood sugar reduction, blood lipid reduction, inflammation resistance, apoptosis resistance, fibrosis resistance, promotion in mitochondrial synthesis and oxidative metabolism, ageing resistance, tumor resistance and the like (Physiol. Rev. 2009, 89, 1025; Nature 2013, 493, 346). More and more evidences show that dysfunction of the AMPK is closely related to occurrence and development of various diseases (J. China Pharm Univ, 2019, 50 (supplement): 101; Nat. Rev. Drug Discov. 2019, 18, 527; Trends Endocrinol. Metab. 2017, 28, 545; Expert Opin. Ther. Targets 2016, 20, 179; Int. J. Immunopath. Ph. 2016, 29, 562; Experimental Neurology 2017, 298, 31). Thus, activation of the AMPK is an important way of preventing and treating various acute or chronic diseases (Nat. Rev. Drug Discov. 2019, 18, 527; J. Med. Chem., 2015, 58, 2; Nature 2013, 493, 346; Experimental Neurology 2017, 298, 31; Biochemical Pharmacology 2010, 80, 1708; Current Drug Targets, 2016, 17, 908). For example, metformin and berberine widely used in the clinical practice exert various clinical efficacies mainly by activating the AMPK (J. Clin. Invest. 2001, 108, 1167). Although an AMPK activator has a wide clinical application prospect, there has been no substantial progress in the field of developing novel, safe and effective AMPK activators so far. Due to the reason for safety or effectivity, very few developed AMPK activators enter the clinical research stage. For example, MK-8722 as a broad-spectrum AMPK activator is discovered to have the side effect of irreversible cardiac hypertrophy on hearts of animals in experiments on rats and monkeys although being capable of reducing blood sugar (Science 2017, 357, 507). Furthermore, clinical experiments of AICAR, as one of the most common AMPK activators, are terminated also due to relatively large toxic and side effect (J. Clin. Pharmacol. 1991, 31, 342). To summarize, it is necessary to develop a novel AMPK activator with high activity and slight toxic and side effect clinically.

A receptor a (ERRα) related to oestrogens encoded by a gene ESRRA (with a gene number in NCBI of Gene ID 2101) is the first discovered orphan nuclear receptor (Front. Endocrinol. 2019, 10, 206). The ERRα is a key transcription factor for mitochondrial synthesis and oxidative metabolism and may promote mitochondrial synthesis and oxidative metabolism through transcriptional regulation (Cell Metabolism 2013, 17, 586). The ERRα further has the effects of inflammation resistance, apoptosis resistance and tissue damage protection and has the action mechanism which mainly comprises three aspects that: (1) A20 is a kind of endogenous anti-inflammatory and anti-apoptosis proteins (Trends in Immunology 2009, 30, 383), and the ERRα may up-regulate the expression level of the A20 proteins (PNAS 2013, 44, 17975; Immunity 2015, 43, 80) and then exerts the effects of inflammation resistance and apoptosis resistance; (2) the ERRα inhibits production of type I interferon by inhibiting TBK1-IRF3 protein-protein interaction and then inhibits the inflammatory responses (PLoS Pathogens 2017, 13, e1006347); and (3) the ERRα may regulate the expression level of genes related to occurrence of autophagy (Autophagy 2019, 14, 152), and then the autophagic protective effect of the organism is exerted. Furthermore, preliminary researches by the inventor show that in in vitro and in vivo models for various hepatic diseases, the stability of the ERRα proteins in hepatocytes or hepatic tissues are significantly weakened, whereas increase in ERRα protein level through gene therapy may be used for preventing and treating the hepatic diseases (such as the non-alcoholic fatty liver disease, non-alcohol steatohepatitis, primary biliary cholangitis, primary sclerosing cholangitis, the alcoholic fatty liver disease, hepatic fibrosis or hepatic cirrhosis) (Chinese Patent Application No. 2020100455303). To summarize, up-regulation of the protein levels of the ERRα may be used for preventing and treating various diseases including the hepatic diseases, the respiratory system diseases, the metabolic disorders, the autoimmune diseases, the cardiovascular and cerebrovascular diseases, the kidney diseases, the central nervous system diseases and the muscular dystrophy (Trends in Endocrinology and Metabolism 2008, 19, 269; Endocrine Reviews 2008, 29, 677). Correlation between the AMPK and the ERRα is that: activation of the AMPK may improve the transcription activity of the ERRα by promoting phosphorylation of PGC-1α (Genes & Development 2016, 30, 1034; Immunity 2015, 43, 80); however, the specific mechanism has not been very clear. Preliminary researches by the inventor show that weakening in stability of the ERRα proteins is the important pathological feature for diseases such as the non-alcohol steatohepatitis (NASH). At present, a compound capable of increasing the protein level of the ERRα has not been reported in any literature.

A disease spectrum of the non-alcoholic fatty liver disease (NAFLD) includes non-alcoholic fatty liver (NAFL), non-alcoholic steatohepatitis (NASH) and NASH- induced hepatic cirrhosis (Science 2011, 332, 1519). At present, the NAFLD has become the second bigger hepatic disease inferior to virus hepatitis. More seriously, severe inflammations and fibrosis of a liver of a patient with the NASH are the common reasons for primary liver cancer and end-stage liver diseases as well as the third most common indication requiring a liver transplant. A recognized pathological mechanism of the NASH is "two-hit" hypothesis (J. Gastroenterol. Hepatol. 2013, 28 (SI1), 68): the "first hit" is liver fat accumulation; and the "second hit" is that oxidative stress and inflammatory cytokine excessive release caused by liver fat accumulation, which causes the liver inflammations and the hepatic fibrosis, and then the hepatic cirrhosis and liver cancers. At present, there has been no effective NASH treating drug clinically, particularly, is short of drugs aiming to "two-hit". More and more researches show that the pathological mechanism of the non-alcoholic fatty liver diseases (particularly, NASH) is closely related to dysfunction of the AMPK (Am. J. Physiol. Endocrinol. Metab. 2016, 311, E730). The metformin and the berberine widely used in the clinical practice exert the efficacy of resisting the non-alcoholic fatty liver diseases (particularly, the NASH) mainly by activating the AMPK (Diabetes Frontier 2013, 24, 418; Experimental and Therapeutic Medicine 2017, 14, 4134). However, the two drugs are both inhibitors for a mitochondrial complex I, and there is a risk of lactic acidosis. In another aspect, down-regulation of the activity of the ERRα may exacerbate symptoms rapamycin-induced fatty liver (Cell Metabolism 2013, 17, 586), which hints that up-regulation of the activity of the ERRα has the preventing and treating effect on the fatty liver diseases (Endocrinology 2018, 159, 2153). The NASH treating drugs recommended to use clinically at present include an insulin sensitizer such as pioglitazone; however, the clinical effects of the above drugs are limited (N. Engl. J. Med. 2010, 362, 1675), and there are risks of fractures, body weight gains and heart failure (Hepatology, 2010, 51, 366). Novel anti-NASH drugs clinically in development include a farnesoid X receptor (FXR) activator (such as obeticholic acid), an acetyl-CoA carboxylase (ACC) inhibitor (such as GS-0976), an apoptosis signal regulating kinase-1 (ASK1) inhibitor (such as, Selonsertib) and the like. However, the curative effects and the safety of the above novel anti-NASH drugs need further verification. To sum up, there is an urgent need for safe and effective novel drugs for resisting the non-alcoholic fatty liver diseases (particularly the NASH) clinically.

The primary biliary cholangitis (PBC) is also known as primary biliary cirrhosis and a kind of organ-specific chronic cholestatic autoimmune liver diseases. Intrahepatic cholestasis, progressive and nonsupurative inflammatory destructions of anti-mitochondrial antibodies and intrahepatic bile ducts in circulating blood, nonsuppurative inflammatory destructions and finally resultant extensive hepatic duct destruction, biliary cirrhosis and even hepatic failure are notable features of this disease. More than 90% of the patients are female, the age at onset is 30-65 years old, and 30-50% of asymptomatic patients are discovered in routine examination generally. At present, approved drugs for treating PBC mainly include ursodesoxycholic acid, the obeticholic acid, glucocorticoid and ciclosporin. However, there are problems in the aspects of both the effectivity and the safety of the above drugs. For example, the obeticholic acid shows significant curative effect in a clinical trial; however, the side effects of increase in level of low density lipoprotein cholesterin (LDL-c) and pruritus of the obeticholic acid may limit clinical application (Lancet 2015, 385, 956).

The primary sclerosing cholangitis (PSC) is also a kind of chronic cholestatic diseases and has the features of progressive inflammations and fibrosis of intra-and extra-hepatic bile ducts which then cause multifocal biliary stricture. Most of patients develop the hepatic cirrhosis, portal hypertension and hepatic dysfunction. The exact pathological mechanism of the PSC is unknown, which may be caused by participation of the following factors: (1) it is hinted that the PSC may have the autoimmune process as the PSC has close relationship to ulcerative colitis (a kind of autoimmune diseases). (2) The inflammatory responses of liver and bile ducts may be induced as bacteria chronically or repeatedly enter portal circulation; and furthermore, colon bacterial or chronic viral infection causes toxic bile acid accumulation and may also cause liver injury. (3) Ischemic injury of the bile ducts may also occur. At present, there has been no effective treatment drug aiming to the PSC.

The alcoholic fatty liver disease (AFLD) is a kind of the hepatic diseases caused by great alcohol consumption for a long term and is one of types of alcoholic liver diseases. The patients have a long history of drinking over 5 years generally. The patients have non-specific clinical symptoms, may be asymptomatic, or have symptoms of right upper quadrant distending pain, anorexia, weakness, loss of weight and the like. At present, there has been no effective treatment drug aiming to the AFLD. Existing treatment means include that: treatment with S-ademetionine may improve the clinical symptoms and biochemical indexes of the patients with the AFLD; polyene phosphatidyl choline has a trend of preventing histological deterioration for the patients with the AFLD; and drugs, including a glycyrrhizin preparation, silymarin drugs, polyene phosphatidyl choline and reduced glutathione, have effects of resisting oxidation and inflammations, protecting liver cell membranes and cell organelles and the like in different degrees and may improve the biochemical indexes of the liver after being clinically applied. It is reported by Zhu et al. that a fibroblast growth factor 21 (FGF21) exerts the efficacy of resisting the AFLD by activating the AMPK (Acta Biochimica et Biophysica Sinica 2014, 46, 1041). Dihydroquercetin exerts the effect of resisting the AFLD by activating the AMPK (Journal of Agricultural and Food Chemistry 2018, 66, 4862). The above research results hint that the AMPK activators may be used for preventing and treating the AFLD.

The hepatic fibrosis refers to intrahepatic connective tissue dysplasia due to various pathological factors. Any liver injury has the process of hepatic fibrosis in the process of liver healing and repair. If injury factors cannot be removed for a long term, development of the hepatic cirrhosis may be caused by long lasting of the fibrosis process. At present, there is still short of effective treatment means for resisting the hepatic fibrosis, and existing treatment methods mainly include: (1) removal of the pathogenic factors aiming to primary diseases, such as treatment of resisting hepatitis B virus, hepatitis C virus and schistosoma and cut out alcohol; and (2) treatment aiming to the hepatic fibrosis self, such as through inhibition of inflammations, lipid peroxidation or hyperplasia and activation of hepatic stellate cells and promotion in collagen degradation. It is reported by Grouix et al. that activation of the AMPK may effectively prevent and treat the hepatic fibrosis (Journal of Pharmacology and Experimental Therapeutics 2018, 367, 71).

The hepatic cirrhosis is a clinically common chronic progressive liver disease and is a kind of diffuse liver injuries caused by long-term or repeated affects by one or more of causes of disease. In China, most of the patients with the hepatic cirrhosis have posthepatitic cirrhosis, and a minority of the patients have alcoholic cirrhosis, the NASH-induced hepatic cirrhosis, cholestatic cirrhosis, schistosomiasis cirrhosis of liver and the like. There are extensive necrosis of hepatocytes, nodular regeneration of survival hepatocytes, connective tissue proliferation and formation of fibrous septa histopathologically, which cause destruction of a liver lobule structure and formation of pseudolobule, and the liver deforms and becomes stiffened gradually to develop the hepatic cirrhosis. The patients may have no obvious symptom due to relatively strong liver compensation in early stage, have main manifestations of hepatic function damages and portal hypertension in later period with multisystem involvement and have complications upper gastrointestinal hemorrhage of hepatic encephalopathy, secondary infection, hypersplenism, ascites, canceration and the like. The hepatic cirrhosis is a kind of liver dysfunction caused by tissue structure disorder, and there has been no radical cure yet. It mainly lies on early discovering and preventing disease progression, particularly, preventing and treating complications of the hepatic cirrhosis. It is reported by Hu et al. that AICAR, as the AMPK activator, may effectively relieve symptoms of the portal hypertension and the hepatic cirrhosis (Journal of Molecular Medicine-JMM 2019, 97, 423).

The common pathological features of the respiratory system diseases, including cough, asthma, trachitis, bronchitis, pneumonia, respiratory distress syndrome, emphysema, chronic obstructive pulmonary disease (COPD), idiopathic pulmonary fibrosis, cystic fibrosis pulmonary disease and rhinitis, are inflammations and airway tissue fibrosis; whereas existing anti-inflammatory drugs aiming to the respiratory system diseases are mainly glucocorticoid drugs (such as budesonide and fluticasone propionate), and these hormone drugs may cause the side effects of osteoporosis, femoral head necrosis, acute lung inflammation, oral candidosis infection and the like. Thus, existing drugs for resisting the respiratory system diseases are far from meeting the clinical requirements, particularly, treatment drugs for respiratory distress syndrome caused by severe pulmonary infection (with a source of infection of novel coronavirus, bacteria and the like) are very limited. Note that knocking out AMPKα1 may worsen the lung inflammatory responses and the emphysema of smoked mice (Tuberculosis and Respiratory Diseases, 2015, 78, 8), which hints that the AMPK has the protection effect on lung inflammatory injury. Furthermore, the AICAR and the metformin, as the AMPK activators, may significantly inhibit the inflammatory responses of respiratory tracts of the smoked mice (Oncotarget 2017, 8, 22513). Furthermore, it is reported by Yuan et al. that the AICAR has the protection effect on epithelial cell apoptosis of the respiratory tracts caused by tobaccos (Biochemical and Biophysical Research Communications 2018, 497, 564). In another aspect, acute pulmonary infection may induce overexpression of type I interferon to cause apoptosis as well as inflammatory injury and fibrosis of lung tissues, and then the diseases get worse, and even the patients die. It is reported by He et al. that the ERRα may inhibit excessive generation of the type I interferon by inhibiting TBK1-IRF3 protein-protein interaction (PLoS Pathogens 2017, 13, e1006347), which hints that improvement in activity of the ERRα may prevent and treat the acute lung inflammations and the respiratory distress syndrome caused by viral or bacteria infection.

The metabolic disorders include the insulin resistance, metabolic syndromes, diabetes and complications thereof, hyperlipidemia, obesity, hyperuricemia, gout and the osteoporosis. It is well known that activation of the AMPK (through exercise, fasting, the AMPK activators or the like) may prevent and treat the above metabolic disorders (Nat. Rev. Drug Discov. 2019, 18, 527). In another aspect, the ERRα may also be taken as a novel drug target for resisting the metabolic disorders by the efficacy of enhancing oxidative metabolism, resisting inflammations and fibrosis, promoting protective autophagy and the like (Acta Pharmacologica Sinica 2015, 36, 51).

The complications of the diabetes are main reasons for disability and death caused by the diabetes and mainly include: (1) macrovascular complications, such as cerebrovascular lesions, cardiovascular lesions and lower extremity vascular lesions (which may cause diabetes ulcer, particularly diabetic foot); (2) microvascular complications, such as kidney lesions and ocular fundus pathology; and (3) neuropathy, including sensory nerves responsible for sense organ, motor nerves for controlling physical activities and autonomic nerves for managing viscera, blood vessels and endocrine function. The complications of the diabetes specifically include: diabetic nephropathy, diabetic cardiomyopathy, the diabetes ulcer (particularly, the diabetic foot), diabetic cerebrovascular diseases, diabetic retinopathy and diabetic neuropathy. The pathological mechanism of the complications of the diabetes is also closely related to dysfunction of the AMPK (Journal of Clinical Investigation 2013, 123, 4888). It is reported in literatures that the AMPK activators may be used for preventing and treating the diabetic nephropathy (Journal of Pharmacology and Experimental Therapeutics 2017, 361, 303; PLoS One 2014, 9, e113398; WO2014086104A1). Activation of the AMPK also have significant curative effect on the diabetic cardiomyopathy (ell Death & Disease 2018, 9, 1; Clinical Science 2017, 131, 187; Biochemical and Biophysical Research Communications 2017, 482, 665; Diabetes 2013, 62, 1270). Furthermore, animal experiments and clinical researches show that the AMPK activators may further be used for preventing and treating the diabetes ulcer (particularly, the diabetic foot) (Expert Opinion on Investigational Drugs 2014, 23, 1253).

The autoimmune diseases include ulcerative colitis, Crohn's disease, systemic lupus erythematosus, familial chilblain lupus, Chagas disease, rheumatoid arthritis, psoriasis, multiple sclerosis, scleroderma , Behcet's disease and Aicardi-Goutières syndromes and have the common pathological mechanism that an innate immune system is continuously activated due to factors of inflammation irritants, genetic defects or the like, then acquired immune responses are induced, and finally, chronic immune inflammatory responses of the organism are caused. More and more evidences show that the AMPK has the efficacy of resisting the autoimmune diseases, and the main action mechanism of resisting the inflammatory responses includes: (1) the important pathological feature of the autoimmune diseases is that excessive apoptosis causes tissue injuries and the fibrosis, whereas the AMPK shows the anti-apoptosis effect on various cells (including endothelial cells) (PNAS 2004, 101, 3329); and furthermore, the AMPK also has the effect of resisting the fibrosis (such as the hepatic fibrosis, pulmonary fibrosis and renal fibrosis) (J. Pharmacol. Exp. Ther. 2018, 367, 71; PLoS ONE 2018, 13(8), e0201692; J. Am. Soc. Nephrol. 2018, 29, 2326); (2) various immune inflammatory responses may cause dysfunction of mitochondria and then promote inflammatory amplification effects of increase in reactive oxygen species (ROS), leakage of mitochondrial DNA, activation of inflammasomes, activation of cGAS-STING signaling pathways and the like, whereas the AMPK may maintain homeostasis of the mitochondria by promoting mitochondrial synthesis and mitophagy and the like and then exerts the efficacy of resisting immune inflammations (Nat. Rev. Drug Discov. 2019, 18, 527); and (3) the AMPK may induce metabolism reprogramming of immune cells (such as macrophages and T cells), that is, promote conversion of cell glycolysis to oxidative metabolism to polarize the immune cells from a pro-inflammatory type (such as M1 macrophages and Th17 cells) to an immunosuppressive type (such as M2 macrophages and regulatory T cells) (Nature 2013, 493, 346) and then exert the immunosuppressive effect. To sum up, the AMPK activators may be used for preventing and treating the autoimmune diseases. In another aspect, functional defects of A20 are closely related to occurrence and development of the autoimmune diseases (Trends in Immunology 2009, 30, 383), whereas the ERRα may exert the effects of resisting inflammations and apoptosis through up-regulation of the expression level of the A20 (Immunity 2015, 43, 80). Thus, drugs capable of up-regulating the protein level of the ERRα are expected to be useful for preventing and treating the autoimmune diseases.

Inflammatory bowel diseases (IBD) are a kind of chronic non-specific inflammatory diseases involving intestinal tracts, mainly including the ulcerative colitis and the Crohn's disease. At present, clinical treatment drugs for the inflammatory bowel diseases include aminosalicylic acid drugs (5-aminosalicylic acid, sulfasalazine and the like), glucocorticoid drugs (hydrocortisone, budesonide and the like), immunosuppressive agents (azathioprine, methotrexate and the like), anti-inflammatory factor monoclonal antibodies (adalimumab, infliximab and the like) and the like. However, existing drugs can partially alleviate the conditions of these diseases only and are difficult to thoroughly cure the diseases. It is reported in literatures that the treatment effect of the metformin on the inflammatory bowel diseases is achieved by activating the AMPK to exert the anti-inflammatory effect (Clinical Science 2018, 132, 1155). The AICAR, as the AMPK activator, (Eur. J. Biochem. 1995, 229, 558) has significant curative effect on the inflammatory bowel diseases by inhibiting innate immunity and acquired immune inflammations (Biochemical Pharmacology 2010, 80, 1708); however, the side effect of the drug is relatively large. To summarize, there is an urgent need for drugs capable of effectively resisting the inflammatory bowel diseases clinically.

The systemic lupus erythematosus (SLE) is one of autoimmune inflammatory connective tissue diseases, typically striking young female and involving multiple organs. Under the interaction of various factors of genetic factors, environmental factors, estrogen level and the like, functions of suppressor T cells are decreased, and B cells are subjected to hyper-proliferation to produce a great amount of autoantibodies; and the autoantibodies are bound to corresponding autoantigens in vivo to form a corresponding immune complex, the immune complex deposits at parts of skin, joints, small blood vessels, glomeruli and the like to cause acute and chronic inflammations and tissue necrosis (such as lupus nephritis), and thus multisystem injury of the organ is caused. The existing treatment drugs include: non-steroid anti-inflammatory drugs (NSAIDS), chloroquine, hydroxychloroquine, the glucocorticoid, cyclophosphamide, azathioprine, the methotrexate, the ciclosporin A, vincristine and the like. However, there are problems in the aspects of both the curative effect and the safety of the above drugs. Researches show that the metformin, as the AMPK activator, exerts the effect of resisting the SLE by inhibiting type I interferon response of CD4 (+) T cells (Journal of Immunology 2019, 203, 338) and inhibiting B cell differentiation (Journal of Immunology 2017, 198, 2661). In another aspect, overexpression of the type I interferon is an important pathological index for the SLE, whereas the ERRα may inhibit production of the type I interferon by inhibiting the TBK1-IRF3 protein-protein interaction (PLoS Pathogens 2017, 13, e1006347). Furthermore, the ERRα may exert the effect of resisting the SLE by up-regulating the expression level of A20 proteins (PNAS 2013, 44, 17975; Immunity 2015, 43, 80) (Trends in Immunology 2009, 30, 383).

The rheumatoid arthritis (RA) is one of the autoimmune diseases, which are characterized by arthromeningitis, and is characterized by polyarticular, symmetrical and invasive arthritis of small joints of hands and feet, often accompanied by abarticular organ involvement and positive serum rheumatoid factors, which may cause joint deformities and dysfunction. Treatment drugs mainly include the non-steroid anti-inflammatory drugs (such as diclofenac), the immunosuppressive agents (such as the methotrexate), the glucocorticoid, biological preparations (such as the infliximab) and the like. Researches show that in peripheral leucocytes of newly diagnosed patients with the RA, the gene expression level of the AMPK is significantly down-regulated (Molecular Biology Reports 2019, 46, 6353), whereas activation of the AMPK also has the treatment effect on the RA (Current Rheumatology Reviews 2019, 15, 116; European Journal of Pharmacology 2019, 852, 179; Frontiers in Immunology 2017, 8, 1135). For example, the treatment effects of phenformin, the metformin and the methotrexate on the RA are at least partially achieved by activation of the AMPK (Dermato-Endocrinology 2013, 5, 252). In another aspect, the expression level of the ERRα in an RA animal mode is down-regulated (Rheumatology 2008, 47, 1785), which hints that afunction of the ERRα plays an important role in the pathological mechanism of the RA.

The psoriasis is one of autoimmune diseases and has the main clinical manifestations of lesions, scales and red plaques of the skin and may cause severe pains or itches, which severely affects the life quality of the patients. Complications of the psoriasis include psoriatic arthritis, nail psoriasis and enthesitis. Immunodeficiencies are considered the main pathological mechanism of the psoriasis, particularly, abnormity of interaction between the innate immune system and the acquired immune system of a human body (Lancet 2015, 386, 983). The innate immune system plays an important role in occurrence of the psoriasis and early disease progression (J. Invest. Dermatol. 2010, 130, 1213). Decrease in activity of the AMPK in psoriatic skin is an important pathological feature of such diseases (The Journal of investigative dermatology 2015, 135, 2732), whereas the metformin and the methotrexate exert the efficacy of resisting the inflammations and the psoriasis by activating the AMPK (Dermato-Endocrinology 2013, 5, 252; British Journal of Dermatology 2018, 179, 818).

The multiple sclerosis (MS) is one of the most common demyelinating diseases of the central nervous system. White matters of the central nervous system in the acute period of this disease are multiple inflammatory demyelinating plaques, mainly occurring on optic nerves, spinal cords and brain stems. Treatment drugs for the white matters of the central nervous system include: gilenya, hormones, interferon, glatiramer acetate, mitoxantrone and the like. Newest researches show that afunction of LKB1 (upstream kinase of the AMPK) is an important risk factor for the MS (Glia 2020, 68, 600), which hints that abnormity in AMPK signaling pathways may be the pathological factor of the MS. The metformin has the curative effect on an MS animal model by activating the AMPK (Journal of Immunology 2009, 182, 8005; DARU-Journal of Pharmaceutical Sciences, DOI: 10.1007/s40199-019-00286-z).

Behcet's disease is one of the autoimmune diseases and belongs to vasculitis. The Behcet's disease may invade multiple organs of the human body, including the oral cavity, the skin, joints, muscles, eyes, blood vessels, the heart, the lung and the nervous system, and has the main manifestations of repeated ulcers of the oral cavity and perineum, skin rash, lower extremity erythema nodosa, eye iritis, esophageal ulcers, small intestinal or colonic ulcers, arthralgia and the like. Treatment drugs for the Behcet's disease include: the glucocorticoid, the methotrexate, colchicine, thalidomide, the azathioprine, the cyclophosphamide, the ciclosporin, mycophenolate mofetil and anti-tumor necrosis factor antagonists; however, the side effects of the above drugs are very large.

The cardiovascular and cerebrovascular diseases include the hypertension, atherosclerosis, arrhythmia, coronary heart diseases, myocardial infarction, myocardial hypertrophy, carditis, the heart failure, pulmonary arterial hypertension, cerebral apoplexy, stroke, cerebral infarction and cerebral edema and have the common pathological mechanism of vascular endothelial dysfunction (increase in endothelial cell apoptosis, inflammatory injuries of endothelial cells and the like) and vascular fibrosis. Researches show that the metformin improves functions of the blood vessels by activating the AMPK and inhibiting endoplastic reticulum stress and then exerts the effect of reducing the blood pressure (Hypertension Research 2019, 42, 960; British Journal of Pharmacology 2017, 174, 2140). Researches by Omura et al. show that the activity of the AMPK in pulmonary arterial hypertension is decreased (Circulation Research 2019, 125, 884), whereas activation of the AMPK may improve the symptoms of the pulmonary arterial hypertension (Molecular and Cellular Biochemistry 2019, 455, 169; Life Sciences 2018, 208, 87). It is reported by Ying et al. that fibroblast growth factors-21 (FGF-21) may improve the vascular endothelial dysfunction caused by the diabetes by activating the AMPK (Cell Death & Disease 2019, 10, 665). The above research results hint that the AMPK activator may be used for preventing and treating various cardiovascular and cerebrovascular diseases.

Polycystic kidney diseases are one of genetic diseases with kidney congenital abnormality. There are two types of the polycystic kidney diseases, wherein one type is autosomal recessive polycystic kidney disease (ARPKD), occurring in the infancy stage, and is relatively rare clinically; and the other type is autosomal dominant polycystic kidney disease (ADPKD), often occurring in young and middle-aged stage or at any age, is a common type of the polycystic kidney diseases and accounts for 8-10% of all causes of end-stage renal failure. There has been no effective method of inhibiting formation and development of cysts aiming to the ADPKD at present. There are methods mainly of symptomatic treatment, prevention and treatment in complications, protection in kidney function and treatment during kidney function reduction. Recently, Tolvaptan, as a vasopressin V2 receptor antagonist, has been approved by American FDA and is used for adult patients with the autosomal dominant polycystic kidney disease (ADPKD) with a risk of rapid disease progression to delay reduction in kidney function. Note that the AMPK plays an important role in the pathological mechanism of the ADPKD (Proc. Natl. Acad. Sci. USA 2011, 108, 2462; J. Am. Soc. Nephrol. 2016, 27, 1437). The disease progression of the ADPKD may be effectively delayed with the metformin, as the AMPK activator, (Proc. Natl. Acad. Sci. USA 2011, 108, 2462) or by dieting (J. Am. Soc. Nephrol. 2016, 27, 1437), which hints that the AMPK activators may be expected to become a novel treatment means for the ADPKD.

The central nervous system diseases include Parkinson diseases, Alzheimer's diseases, a-synucleinopathy, depression, amyotrophic lateral sclerosis, fibromyalgia syndrome, neuralgia, Down's syndrome, Hallervorden-Spatz diseases, Huntington's diseases and Wilson's disease and have the common pathological mechanism of inflammatory responses of the nervous system.

Ginsenoside Ro (I, CAS: 34367-04-9), asperosaponin VI (II, CAS: 39524-08-8), calunduloside E (III, CAS: 26020-14-4), kochioside Ic (IV, CAS: 96990-18-0), asiaticoside (V, CAS: 16830-15-2), madecassoside (VI, CAS: 34540-22-2), saikosaponin A (VII, CAS: 20736-09-8), saikosaponin D (VIII, CAS: 20874-52-6) and reinioside C (IX, CAS:167324-07-4), α-hederin (X, CAS: 27013-91-8), hederacoside C (XI, CAS: 14216-03-6), ziyuglycoside I (XII, CAS: 35286-58-9), ziyuglycoside II (XIII, CAS: 35286-59-0), asperosaponin C (XIV, CAS: 60252-11-1), β-hederin (XV, CAS: 35790-95-5) and hederin A2 (XVI, CAS: 3391-80-8) are all natural pentacyclic triterpenoid saponin and have various biological activities (Natural Product Reports 2011, 28, 543; Archives of Pharmaceutical Research, 1995, 18(3), 164). However, the prevention and treatment effects of the above pentacyclic triterpenoid saponin on the hepatic diseases, the inflammatory bowel diseases, the respiratory system diseases, the metabolic disorders, the kidney diseases, the cardiovascular and cerebrovascular diseases, the autoimmune diseases and the central nervous system diseases are not very clear.

### Summary of the Invention

An objective of the present invention is to provide a use of pentacyclic triterpenoid saponin represented by formulas (I)-(XVI) or pharmaceutically acceptable salts or solvates thereof in preparing a drug for preventing or treating AMPK and/or ERRα-mediated diseases.

The present invention further provides a drug composition for preventing and treating the AMPK and/or ERRa-mediated diseases.

The technical solution is as follows: to achieve the above objective, the use of the pentacyclic triterpenoid saponin represented by the formulas (I)-(XVI) of the present invention or the pharmaceutically acceptable salts or solvates thereof in preparing the drug for preventing or treating the AMPK and/or ERRa-mediated diseases is: 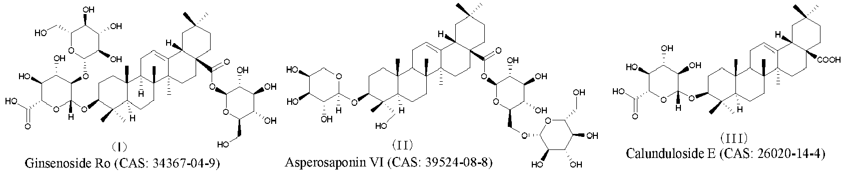 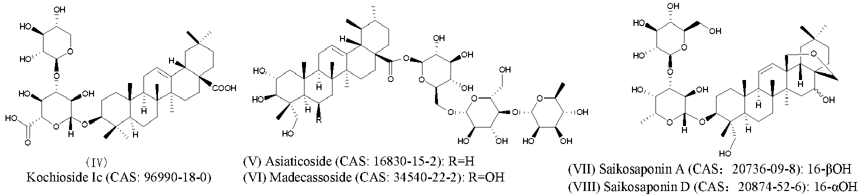 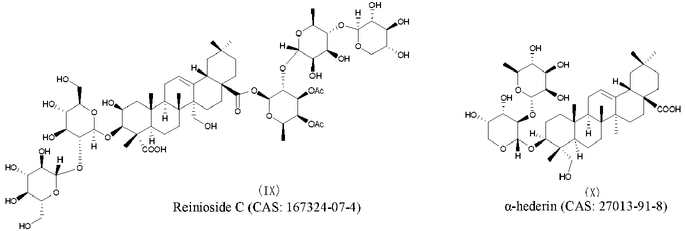 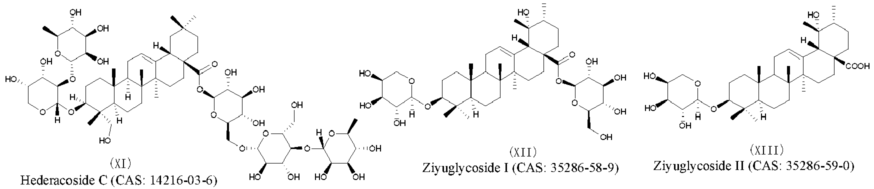 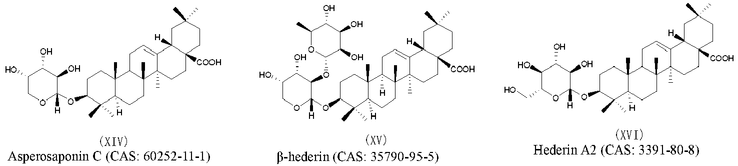 wherein one or more of compounds represented by the formulas (I)-(XVI) may be arbitrarily selected for preparing the drug for preventing or treating the AMPK and/or ERRa-mediated diseases.

The above pentacyclic triterpenoid saponin includes: ginsenoside Ro (I, CAS: 34367-04-9), asperosaponin VI (II, CAS: 39524-08-8), calunduloside E (III, CAS: 26020-14-4), kochioside Ic (IV, CAS: 96990-18-0), asiaticoside (V, CAS: 16830-15-2), madecassoside (VI, CAS: 34540-22-2), saikosaponin A (VII, CAS: 20736-09-8), saikosaponin D (VIII, CAS: 20874-52-6), reinioside C (IX, CAS: 167324-07-4), α-hederin (X, CAS: 27013-91-8), hederacoside C (XI, CAS: 14216-03-6), ziyuglycoside I (XII, CAS: 35286-58-9), ziyuglycoside II (XIII, CAS: 35286-59-0), asperosaponin C (XIV, CAS: 60252-11-1), β-hederin (XV, CAS:35790-95-5) and hederin A2 (XVI, CAS: 3391-80-8).

In some implementations, any one of the compounds represented by the formulas (I)-(XVI) may be a plant extract with a content of 10-99% (by weight); preferably, the content of any compound is 20-95%; and more preferably, the content of any compound is 30-90%.

In some implementations, any one of the compounds represented by the formulas (I)-(XVI) may be a product prepared with semi-synthesis with a content of 95% or above (by weight); and preferably, the content of any compound is 98% or above.

The inventor has surprisingly found that the compounds represented by the formulas (I)-(XVI) have very strong AMPK activation activity which is significantly superior to recognized AMPK activators such as metformin and AICAR. Furthermore, the compounds represented by the formulas (I)-(XVI) may significantly up-regulate the protein level of ERRα.

Preferably, the use of the pentacyclic triterpenoid saponin represented by the formulas (I)-(XIII) or the pharmaceutically acceptable salts or solvates thereof in preparing the drug for preventing or treating the AMPK-mediated diseases is: 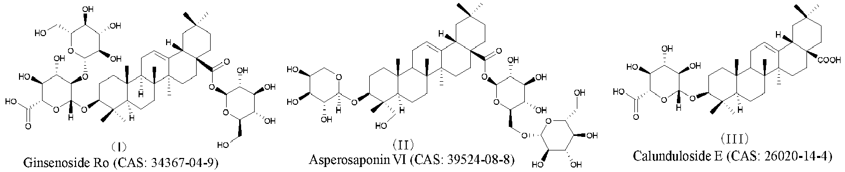 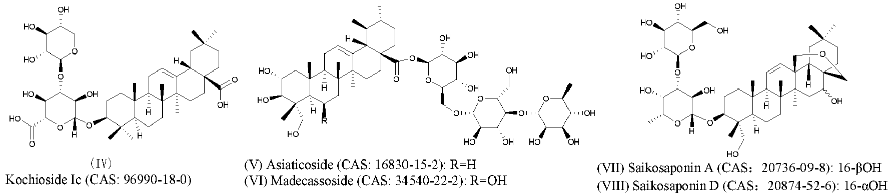 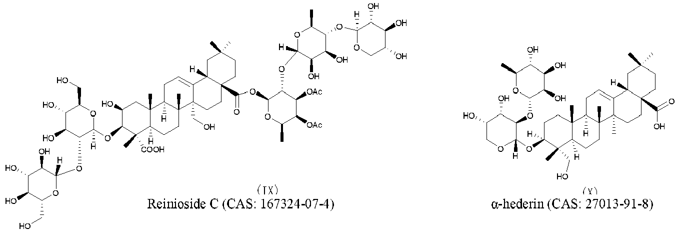 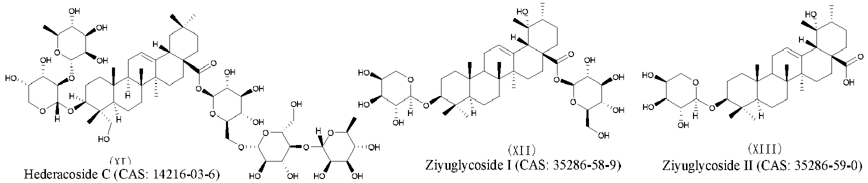 wherein one or more of compounds represented by the formulas (I)-(XIII) may be arbitrarily selected for preparing the drug for preventing or treating the AMPK and/or ERRa-mediated diseases.

The AMPK and/or ERRa-mediated diseases include hepatic diseases, respiratory system diseases, metabolic disorders, autoimmune diseases, cardiovascular and cerebrovascular diseases, kidney diseases, central nervous system diseases or muscular dystrophy.

In some implementations, the hepatic diseases include: non-alcoholic fatty liver, non-alcoholic steatohepatitis, alcoholic fatty liver disease, hepatic fibrosis, hepatic cirrhosis, primary biliary cholangitis or primary sclerosing cholangitis.

In some embodiments, the respiratory system diseases include: cough, asthma, chronic obstructive pulmonary disease, bronchitis, pneumonia, respiratory distress syndrome, emphysema, idiopathic pulmonary fibrosis, cystic fibrosis pulmonary disease, allergic rhinitis or chronic rhinitis.

In some implementations, the metabolic disorders include: insulin resistance, metabolic syndromes, diabetes and complications thereof, hyperlipidemia, obesity, hyperuricemia, gout or osteoporosis.

In some implementations, the autoimmune diseases include: ulcerative colitis, Crohn's disease, systemic lupus erythematosus, rheumatoid arthritis, psoriasis, multiple sclerosis or Behcet's disease.

In some implementations, the cardiovascular and cerebrovascular diseases include: hypertension, atherosclerosis, arrhythmia, coronary heart diseases, myocardial infarction, myocardial hypertrophy, carditis, heart failure, pulmonary arterial hypertension, cerebral apoplexy, stroke, cerebral infarction or cerebral edema.

In some implementations, the kidney diseases include: autosomal recessive polycystic kidney disease or chronic nephritis.

In some implementations, the central nervous system diseases include: Parkinson diseases, Alzheimer's diseases, a-synucleinopathy, depression, amyotrophic lateral sclerosis, fibromyalgia syndrome, neuralgia, Down's syndrome, Hallervorden-Spatz diseases, Huntington's diseases or Wilson's disease.

In some implementations, the muscular dystrophy is Duchenne muscular dystrophy.

As a preferred solution, the hepatic diseases, for which the compounds represented by the formulas (I)-(XVI) or the pharmaceutically acceptable salts or solvates thereof of the present invention are suitable, are the non-alcoholic steatohepatitis, the alcoholic fatty liver disease, the primary biliary cholangitis or the primary sclerosing cholangitis.

As a preferred solution, the respiratory system diseases, for which the compounds represented by the formulas (I)-(XVI) or the pharmaceutically acceptable salts or solvates thereof of the present invention are suitable, are the cough, the asthma, the chronic obstructive pulmonary disease, the bronchitis, the pneumonia, the idiopathic pulmonary fibrosis or the cystic fibrosis pulmonary disease.

As a preferred solution, the metabolic disorders, for which the compounds represented by the formulas (I)-(XVI) or the pharmaceutically acceptable salts or solvates thereof of the present invention are suitable, complications of diabetes or hyperlipidemia. The complications of the diabetes include diabetic nephropathy, diabetic cardiomyopathy or diabetes ulcer (particularly diabetic foot).

As a preferred solution, the autoimmune diseases, for which the compounds represented by the formulas (I)-(XVI) or the pharmaceutically acceptable salts or solvates thereof of the present invention are suitable, are the ulcerative colitis, the Crohn's disease or the psoriasis.

As a preferred solution, the cardiovascular and cerebrovascular diseases, for which the compounds represented by the formulas (I)-(XVI) or the pharmaceutically acceptable salts or solvates thereof of the present invention are suitable, are hypertension, atherosclerosis, coronary heart diseases, myocardial infarction, carditis, heart failure, pulmonary arterial hypertension or cerebral apoplexy.

As a preferred solution, the kidney diseases, for which the compounds represented by the formulas (I)-(XVI) or the pharmaceutically acceptable salts or solvates thereof of the present invention are suitable, are the autosomal dominant polycystic kidney disease.

As a preferred solution, the central nervous system diseases, for which the compounds represented by the formulas (I)-(XVI) or the pharmaceutically acceptable salts or solvates thereof of the present invention are suitable, are Parkinson diseases, Alzheimer's diseases.

In some implementations, the pharmaceutically acceptable salts of the compounds represented by the formulas (I)-(XVI) are metal ions, pharmaceutically acceptable amine and ammonium ions or salts formed by choline, wherein the metal ions include, but not limited to, sodium ions, potassium ions, calcium ions or other ions; and the pharmaceutically acceptable amine includes, but not limited to, ethylendiamine, tromethamine and the like.

The present invention further provides the drug composition for preventing and treating the AMPK and/or ERRa-mediated diseases. The drug composition contains a therapeutically effective amount of any or more of the compounds represented by formulas (I)-(XVI) or pharmaceutically acceptable salts or solvates thereof as active ingredients and pharmaceutically acceptable adjuvants.

The adjuvants, capable of arbitrarily mixed in the drug composition of the present invention, may be changed based on a dosage form, an administration form and the like. The adjuvants include an excipient, an adhesive, a disintegrating agent, a lubricant, a corrigent, a flavoring agent, a coloring agent and a sweetener. Administration routes of the drug composition may be oral, sublingual, transdermal, intramuscular or subcutaneous, skin mucosal, intravenous or the like. The drug composition may be of a pharmacally conventional preparation form including capsule, powder, a tablet, granules, a pill, an injection, a syrup, oral liquid, an inhalant, a cream, an ointment, a suppository or a patch.

In the aspect of the action mechanism of preventing and treating the fatty liver diseases (particularly, NASH), the compounds represented by the formulas (I)-(XVI) may inhibit synthesis of fatty acid and cholesterol of liver by activating the AMPK so as to lower the contents of triglyceride (TG) and low density lipoprotein cholesterin (LDL-c) of the liver, and then the symptom ("first hit") of fat accumulation of the liver, as one of the main pathological features of the fatty liver diseases (particularly, the NASH) may be improved. Furthermore, the compounds represented by the formulas (I)-(XVI) may increase the protein level and the transcription function of the ERRα by activating the AMPK, then exert the effects of resisting liver inflammations and hepatic fibrosis and thus may resist the "second hit" of the NASH (the liver inflammations and the hepatic fibrosis). Based on this, the compounds represented by the formulas (I)-(XVI) may prevent and treat the fatty liver diseases, including the non-alcoholic fatty liver, the non-alcoholic steatohepatitis as well as fatty liver-induced hepatic fibrosis and hepatic cirrhosis.

In the aspect of the action mechanism of preventing and treating the primary biliary cholangitis and the primary sclerosing cholangitis, the compounds represented by the formulas (I)-(XVI) may increase the protein level and the transcription function of the ERRα by activating the AMPK and then exert the following efficacy that: (1) the ERRα may regulate the expression level of genes related to autophagy ad then may inhibit inflammatory responses and fibrosis with protective autophagy and mitophagy; and (2) the ERRα has the effect of resisting apoptosis.

In the aspect of the action mechanism of preventing and treating the inflammatory bowel diseases, it is reported in literatures (Clinical Science 2018, 132, 1155; Biochemical Pharmacology 2010, 80, 1708) that except for inhibiting the intestinal inflammation responses, the AMPK activator may exert the efficacy of resisting the inflammatory bowel diseases by improving the functions of tight junctions of intestinal tracts and an intestinal epithelial cell barrier (Frontiers in Pharmacology 2018, DOI: 10.3389/fphar.2018.00761; Tissue Barriers 2018, 6, 1; Journal of Gastroenterology and Hepatology 2019, 34, 186). Based on this, the compounds represented by the formulas (I)-(XVI) may inhibit the intestinal inflammation responses on one hand, may further improve the functions of tight junctions of intestinal tracts and an intestinal epithelial cell barrier on the other hand by activating the AMPK and then exert the efficacy of resisting the inflammatory bowel diseases (particularly, the ulcerative colitis or the Crohn's disease).

In the aspect of the action mechanism of preventing and treating the respiratory system diseases, some researches show that the AMPK may alleviate the conditions of the cough, the asthma, the chronic obstructive pulmonary disease, lung infectious disease and lung tissue injuries of the pulmonary fibrosis (Acta Pharmaceutica Sinica 2014, 49, 1089). For example, in an OVA-induced mouse asthma model, the metformin, as the AMPK activator may significantly alleviate the conditions of airway inflammations, eosinophil infiltration and airway remodeling and may lower the oxidative stress level of lung tissues of asthmatic mice (Biochemical Pharmacology 2012, 84, 1660). Based on this, the compounds represented by the formulas (I)-(XVI) may prevent and treat the respiratory system diseases, including the chronic obstructive pulmonary disease, the asthma, the idiopathic pulmonary fibrosis, the cystic fibrosis pulmonary disease and the allergic rhinitis, by activating the AMPK.

In the aspect of the action mechanism of preventing and treating the complications of the diabetes, some literatures report that the AMPK activator exerts the efficacy of resisting diabetic nephropathy and diabetic cardiomyopathy through the mechanism of resisting the inflammations, the fibrosis, the apoptosis and the like (Journal of Pharmacology and Experimental Therapeutics 2017, 361, 303; PLoS One 2014, 9, e113398; WO2014086104A1; Cell Death & Disease 2018, 9, 1; Clinical Science 2017, 131, 187; Biochemical and Biophysical Research Communications 2017, 482, 665; Diabetes 2013, 62, 1270). Furthermore, functions of regeneration promotion and the like of the AMPK activators (Human Molecular Genetics 2016, 25, 3178) may facilitate promotion in epidermal wound healing (Aging Cell 2017, 16, 1083) and then may prevent and treat the diabetes ulcer (particularly, the diabetic foot) (Expert Opinion on Investigational Drugs 2014, 23 (9), 1253). To sum up, the compounds represented by the formulas (I)-(XVI) may prevent and treat the complications of the diabetes, including the diabetic nephropathy, the diabetic cardiomyopathy and the diabetes ulcer, by activating the AMPK.

In the aspect of the action mechanism of preventing and treating the polycystic kidney diseases, some literatures report that over-activation of cystic fibrosis transmembrane conductance regulators (CFTR) and mammalian targets of rapamycin (mTOR) play a key role in the pathological mechanism of the polycystic kidney diseases (particularly, the autosomal recessive polycystic kidney disease) (Proc. Natl. Acad. Sci. USA 2011, 108, 2462; J. Am. Soc. Nephrol. 2009, 20, 2493), and the metformin, as the AMPK activator, inhibits the CFTR and the mTOR through phosphorylation and then may delay production of the kidney cysts (Proc. Natl. Acad. Sci. USA 2011, 108, 2462). Further researches discover that similar to tumor cells, for polycystic kidney epithelial cells, ATP is mainly produced through glycolytic enzyme, an ATP/AMP ratio in the cells is increased, the activity of the AMPK as an energy sensor is weakened, the activity of the mTOR is strengthened, and cell proliferation is significantly strengthened (Nat. Med. 2013, 19(4): 488). Based on this, the compounds represented by the formulas (I)-(XVI) may inhibit the CFTR and the mTOR by activating the AMPK and then may prevent and treat the polycystic kidney diseases (particularly, the autosomal recessive polycystic kidney disease).

Furthermore, the inventor discovers that the compounds represented by the formulas (I)-(XVI) may further up-regulate the protein level of the ERRα and strengthen the transcription function of the ERRα.

To sum up, the compounds represented by the formulas (I)-(XVI) or the pharmaceutically acceptable salts or solvates thereof may be used for preparing the drug for preventing or treating the AMPK and/or ERRa-mediated diseases, particularly, preparing the drug for preventing or treating the non-alcoholic fatty liver, the non-alcoholic steatohepatitis, the alcoholic fatty liver disease, the hepatic fibrosis, the hepatic cirrhosis, the primary biliary cholangitis, the primary sclerosing cholangitis, the ulcerative colitis, the Crohn's disease, the chronic obstructive pulmonary disease, the asthma, the idiopathic pulmonary fibrosis, the cystic fibrosis pulmonary disease, the diabetic nephropathy, the diabetic cardiomyopathy, the diabetes ulcer and the autosomal dominant polycystic kidney disease.

In the drug for preventing and treating the AMPK and/or ERRa-mediated diseases of the present invention, the amount of any or more of the compounds represented by formulas (I)-(XVI) or the pharmaceutically acceptable salts or solvates thereof may be properly changed based on an age, a weight and a symptom of a patient, an administration route and the like. When adults (about 60kg) are orally administrated, the administration dosage of any or more of the compounds represented by formulas (I)-(XVI) or the pharmaceutically acceptable salts or solvates thereof is preferably 1-500 mg per time, more preferably, 5-60 mg per time, and administration is conducted for 1-3 times per day. This dosage range may also be deviated from according to different disease conditions or different dosage forms.

The present invention has the beneficial effects that: compared with the prior art, the present invention has the following advantages:
(1) good safety: the compounds represented by formulas (I)-(XVI) are active ingredients of common Chinese herbal medicines such as ginseng, radix dipsaci, *Calendula officinalis,* fructus kochiae, asiatic pennywort herb, radix bupleuri, *Polygala fallax hemsl, Caulis et Folium Hederae Sinensis* and radix sanguisorbae and have very good safety.
(2) Definite curative effect: the compounds represented by the formulas (I)-(XVI) have very good curative effect on the AMPK and/or ERRa-mediated diseases, particularly, the non-alcoholic steatohepatitis, the alcoholic fatty liver disease, the hepatic fibrosis, the hepatic cirrhosis, the primary biliary cholangitis, the primary sclerosing cholangitis, the ulcerative colitis, the Crohn's disease, the chronic obstructive pulmonary disease, the asthma, the idiopathic pulmonary fibrosis, the cystic fibrosis pulmonary disease, the diabetic nephropathy, the diabetic cardiomyopathy, the diabetes ulcer and the autosomal dominant polycystic kidney disease; and the curative effect is equivalent to and even better than that of the existing treatment drugs.
(3) Clear action mechanism: the compounds represented by the formulas (I)-(XVI) have very strong activation activity to the AMPK, may up-regulate the protein level of the ERRα and may strengthen the transcription function of the ERRα. The inventor finds that the compounds as represented by the formulas (I)-(XVI) may exert the curative effect on the AMPK and/or ERRa-mediated diseases by activating an AMPK-ERRα axis; and the AMPK activation activity is significantly superior to the recognized AMPK activator such as the metformin and the AICAR.

### Brief Description of the Drawings

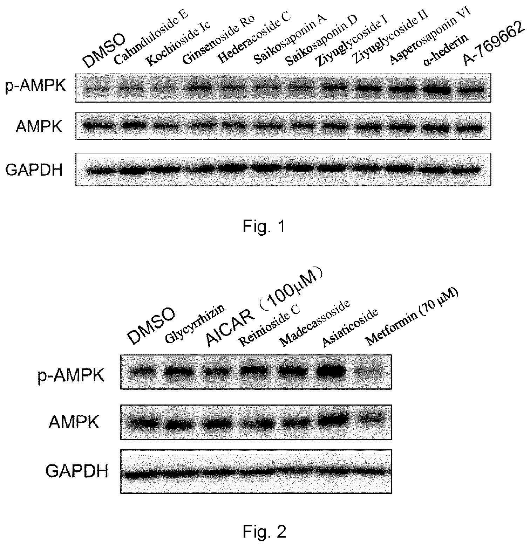

Fig. 1 is an activation activity effect diagram of pentacyclic triterpenoid saponin on AMPK in Huh-7 cells (detected by Western Blot);
Fig. 2 is an activation activity effect diagram of pentacyclic triterpenoid saponin on AMPK in Huh-7 cells (detected by Western Blot);
Fig. 3 is an influence diagram of pentacyclic triterpenoid saponin on AMPK downstream signaling pathways (detected by Western Blot);
Fig. 4 is an influence diagram of ginsenoside Ro on serum lipid (TG, TC, LDL-c and HDL-c) level of high fat diet (HFD)-induced NASH model rats (n=8; compared with an HFD model group, *p<0.05, **p < 0.01, ***p < 0.001; and compared with a CHOW control group, ###p < 0.001);
Fig. 5 is an influence diagram of ginsenoside Ro on liver lipid (TG and TC) level of high fat diet (HFD)-induced NASH model rats (n=8; compared with an HFD model group, *p<0.05, **p < 0.01, ***p < 0.001; and compared with a CHOW control group, ###p < 0.001);
Fig. 6 is an influence diagram of ginsenoside Ro on mRNA expression levels of inflammatory factors (TNFa, IL-6 and IL-1) in high fat diet (HFD)-induced NASH model rats (n=8; compared with an HFD model group, **p < 0.01, ***p < 0.001; and compared with a CHOW control group, ###p < 0.001);
Fig. 7 is an influence diagram of ginsenoside Ro on mRNA expression levels of cell factors (TGF and -SMA) related to fibrosis in livers of high fat diet (HFD)-induced NASH model rats (n=8; compared with an HFD model group, **p < 0.01, ***p < 0.001; and compared with a CHOW control group, ###p < 0.001);
Fig. 8 is an influence diagram of ginsenoside Ro on levels of liver enzymes (ALT and AST) in serum of CCI₄-induced hepatic fibrosis model mice (n=8; compared with a CCl₄ model group, *p < 0.05, **p < 0.01; and compared with a control group, ###p < 0.001);
Fig. 9 is an influence diagram of ginsenoside Ro on mRNA expression levels of cell factors (TGF, -SMA, MMP2 and collagen 1) related to fibrosis in livers of CCl₄-induced hepatic fibrosis model mice (n=8; compared with a CCl₄ model group, *p < 0.05, **p < 0.01, ***p < 0.001; and compared with a control group, ###p < 0.001);
Fig. 10 is an influence diagram of ziyuglycoside II on disease activity indexes (DAI) and colon lengths of ulcerative colitis model mice modeled with dextran sulphate sodium salt (DSS) (compared with the model group, ** P<0.01, *** P<0.001);
Fig. 11 is an influence diagram of ziyuglycoside II on colon histopathology of ulcerative colitis model mice modeled with dextran sulphate sodium salt (DSS);
Fig. 12 is an influence diagram of ziyuglycoside II on inflammatory factors (TNF- and IL-1), reactive oxygen species (ROS) and proteins related to tight junctions (ZO-1, Claudin-1 and Occludin) in colon tissues of the ulcerative colitis model mice modeled with dextran sulphate sodium salt (DSS) (compared with a model group, * P<0.05, ** P<0.01, *** P<0.001);
Fig. 13 is an influence diagram of ziyuglycoside II on weights and colon lengths of Crohn's disease model mice modeled with 2, 4, 6-trinitrobenzene sulfonic acid (TNBS) (compared with the model group, * P<0.05, *** P<0.001);
Fig. 14 is an influence diagram of ziyuglycoside II on inflammatory factors (TNF- and IL-1) and myeloperoxidase (MPO) in colon tissues of Crohn's disease model mice modeled with 2, 4, 6-trinitrobenzene sulfonic acid (TNBS) (compared with the model group, ** P<0.01, *** P<0.001);
Fig. 15 is an inhibit effect diagram of asperosaponin VI on polycystic kidney epithelial cell proliferation (detected with a CCK-8 method); and
Fig. 16 is an influence diagram of asperosaponin VI on signal molecules related to AMPK and proliferation of polycystic kidney epithelial cells (detected by Western Blot).

### Detailed Description of the Invention

The present invention will be further described below in detail in combination with the accompanying drawings and the embodiments. The present invention is not limited to these embodiments. One of skill in the art may properly improve process parameters in view of the disclosure herein. Of particular note, all similar substitutes and modifications are apparent to one of skill in the art and are considered to be included within the present invention.

Compounds represented by formulas (I)-(XVI) in the embodiments of the present invention may be prepared with extraction, separation and purification from Chinese herbal medicines such as ginseng, radix dipsaci, *Calendula officinalis,* fructus kochiae, asiatic pennywort herb, radix bupleuri, *Polygala fallax hemsl, Caulis et Folium Hederae Sinensis* and radix sanguisorbae with reference to methods in literatures (Chinese Patent No. CN102125570B; Natural Product Reports 2011, 28, 543); may also be subjected to semi-synthetic preparation with reference to methods in literatures (Synlett 2004, 2, 259; Natural Product Reports 2011, 28, 543; Arch. Pharm. Chem. Life Sci. 2015, 348, 615); and may also be commercially purchased. The purity of each compound commercially purchased or prepared with semi-synthesis is 95% or above.

### Embodiment 1

### Synthesis of asperosaponin C (XIV, CAS: 60252-11-1)

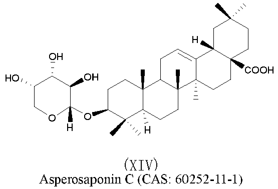

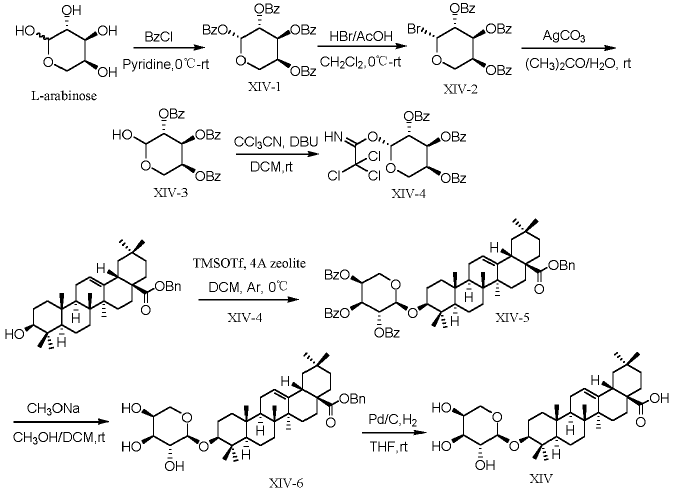

L-arabinose (1 g, 6.66 mmol) was dissolved into pyridine (20 mL), and benzoyl chloride (4.6 mL, 39.97 mmol) was slowly dripped into an ice-water bath for overnight reaction at room temperature. The reaction was monitored with TLC; ethyl acetate (15 mL) was added to dilute a reaction liquid after the reaction was completed; the reaction liquid was washed with water (20 mL), a 1N aqueous hydrochloric acid solution (20 mL), an aqueous saturated NaHCO₃ solution (20 mL) and saturated NaCl (20 mL) in sequence; an organic layer was dried with anhydrous sodium sulfate, concentrated and purified by silica gel chromatography (petroleum ether: ethyl acetate=5: 1) to obtain a compound XIV-1 (white solids, 3.2 g, yield: 84%).

The compound XIV-1 (3.2 g, 5.65 mmol) was dissolved into dichloromethane (20 mL), a solution of 35% HBr in acetic acid (3.5 mL) was added under the ice-water bath, stirring was conducted at the room temperature for reaction, and the reaction was monitored with with TLC. After the reaction was completed, a reactant was washed with the water (20 mL), the aqueous saturated NaHCO3 solution (20 mL) and the saturated NaCl (20 mL) in sequence; an organic layer was dried with the anhydrous sodium sulfate and concentrated to obtain a compound XIV-2. The compound XIV-2 was dissolved into acetone/water (25: 1, 26 mL), silver carbonate (1.85 g, 6.71 mmol) was added, and stirring was conducted at the room temperature for reaction overnight. After the reaction was completed, suction filtration was conducted with diatomite, and a filtered product was subjected to concentration and purified by silica gel chromatography (petroleum ether: ethyl acetate=3: 1) to obtain a compound XIV-3 (white solids, 950 mg, two-step yield: 37%).

The compound XIV-3 (950 mg, 2.05 mmol) was dissolved into anhydrous dichloromethane (15 mL), and trichloroacetonitrile (2.2 mL, 21.94 mmol) and DBU (166 µL, 1.11 mmol) were added for reaction for 3h at the room temperature. After the reaction was completed, a reaction liquid was directly concentrated and purified by silica gel chromatography (petroleum ether: ethyl acetate=3: 1) to obtain a compound XIV-4 (white solids, 1.05 g, yield: 84%).

Benzyl oleanolate (1 g, 1.829 mmol) and the compound XIV-4 (1.33 g, 2.192 mmol) were dissolved into the anhydrous dichloromethane (15 mL), a 4A zeolite (1.9 g) was added, trimethylsilyl trifluoromethanesulfonate (2.5 µL, 0.013 mmol) was added in the ice-water bath, and stirring was conducted for reaction overnight at the room temperature in an argon protection environment. After the reaction was completed, suction filtration was conducted, the zeolite was removed, and concentration was conducted. A residue was purified by silica gel chromatography (petroleum ether: ethyl acetate=10: 1) to obtain a compound XIV-5.

The compound XIV-5 was dissolved into a mixed solution of methanol and the dichloromethane (methanol: dichloromethane=2:1, 24 mL); sodium methoxide (119 mg, 2.20 mmol) was added; reaction was conducted for 5 h at the room temperature; DOWEX50WX2-100 cation exchange resin was added to adjust a pH value of a reaction liquid to be 7; suction filtration was conducted; the resin was removed; concentration was conducted; and a residue was purified by silica gel chromatography (dichloromethane: methanol=10:1) to obtain a compound XIV-6 (white solids, 424 mg, two-step yield: 34%).

The compound XIV-6 (289 mg, 0.426 mmol) was dissolved into tetrahydrofuran (15 mL), 10% Pd/C (30 mg) was added, a hydrogen balloon was added, and stirring was conducted at the room temperature for reaction overnight. After the reaction was completed, a solution was spun dried, diethyl ether (15 mL) was added for stirring overnight, suction filtration was conducted, and a filter cake was washed with the diethyl ether to obtain a compound XIV (white solids, 158 mg, yield: 63%): ¹H NMR (300 MHz, DMSO-*d*₆) ¹H-NMR *(pyridine-ds)* δ:5.47 (br s, 1H), 4.77 (d, 1H, J=7.0), 4.43 (t, 1H, J=8.7), 4.31 (m, 2H), 4.16 (dd, 1H, J=8.7, 3.1), 3.83 (m, 1H), 3.37-3.27 (m, 2H), 1.29, 1.27, 1.00, 0.99, 0.94, 0.93, 0.83 (s, 7×3H, CH3); ESI-MS: 611.5 [(M+Na)⁺]. HRMS: m/z 587.3962 [M-H]⁻ ([C₃₅H₅₅O₇] = 587.3953).

### Embodiment 2

### Synthesis of -hederin (XV, CAS: 35790-95-5)

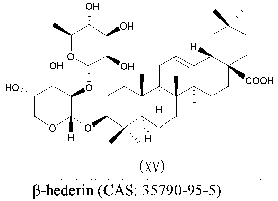

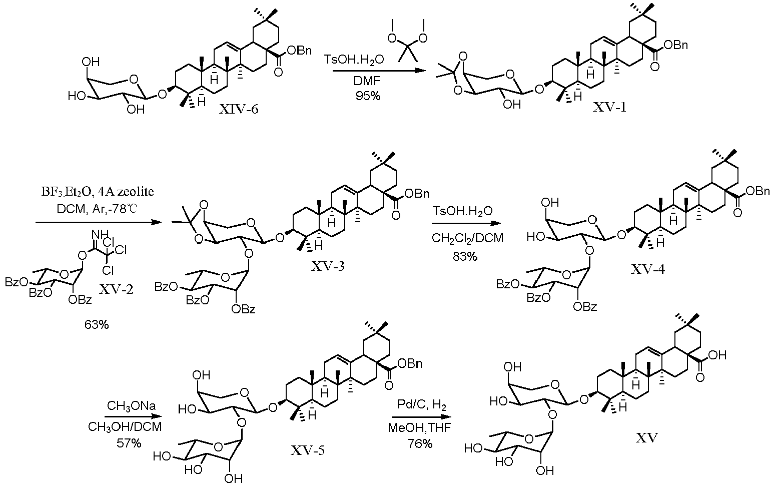

The compound XIV-6 (424 mg, 0.624 mmol) prepared with reference to Embodiment 1 was dissolved into anhydrous N, N-dimethylformamide (15 mL); toluenesulfonic acid (18 mg, 0.095 mmol) and 2, 2-dimethoxypropane (230 µL, 1.872 mmol) were added in an ice-water bath; and reaction was conducted for 5 h at room temperature. After the reaction was completed, 0.3 mL of triethylamine was dripped for quenching reaction, 20 mL of ethyl acetate was added to dilute a reaction liquid, the reaction liquid was washed with water (30 mL) and a saturated salt solution (30 mL) separately, and an organic layer was dried with anhydrous sodium sulfate and was concentrated. A residue was purified by silica gel chromatography (petroleum ether: ethyl acetate=5: 1) to obtain a compound XV-1 (white solids, 426 mg, yield: 95%).

A compound XV-2 was prepared with reference to methods in literatures (Genetics & Molecular Research, 2016, 15, doi: 10.4238/gmr.15038998). The compound XV-1 (150 mg, 0.209 mmol), the compound XV-2 (169 mg, 0.272 mmol) and a 4Azeolite (225 mg) were added in anhydrous dichloromethane (15 mL), a mixed solution was stirred for 40 min at the room temperature and then was cooled to -78°C, a boron trifluoride-diethyl ether complex (18 µL, 0.146 mmol) was added at this temperature, and reaction was conducted for 3 h at -78°C. After the reaction was completed, 0.2 mL of triethylamine was dripped for quenching reaction, and suction filtration and concentration were conducted. A residue was purified by silica gel chromatography (petroleum ether: ethyl acetate=8: 1) to obtain a compound XV-3 (colorless oily liquid, 154 mg, yield: 63%).

The compound XV-3 (150 mg, 0.128 mmol) was dissolved into a mixed solvent of the dichloromethane and methanol (15 mL, dichloromethane: methanol=1: 2), toluenesulfonic acid was added (24 mg, 0.128 mmol), stirring was conducted for reaction at the room temperature, and the reaction was monitored with TLC. After the reaction was completed, a few drops of the triethylamine were dripped to terminate the reaction, a solvent was spun dried, and a residue was purified by silica gel chromatography (petroleum ether: ethyl acetate=2: 1) to obtain a compound XV-4 (white solids, 120 mg, yield: 83%).

The compound XV-4 was dissolved into a mixed solvent of the methanol and the dichloromethane (methanol: dichloromethane=2: 1, 15 mL), and sodium methoxide (23 mg, 0.426 mmol) was added for reaction for 5 h at the room temperature. After the reaction was completed, DOWEX50WX2-100 cation exchange resin was added to adjust a pH value of reaction liquid to be 7 and was removed by suction filtration, and concentration was conducted. A residue was purified by silica gel chromatography (dichloromethane: methanol=20: 1) to obtain a compound XV-5 (white solids, 50 mg, yield: 57%).

The compound XV-5 (50 mg) was dissolved into a mixed solvent of the methanol and tetrahydrofuran (methanol: tetrahydrofuran=1: 1, 15 mL), 10% Pd/C (5 mg) was added, a hydrogen balloon was added, and stirring was conducted at the room temperature for reaction overnight. After the reaction was completed, a reactant was subjected to suction filtration with diatomite, a filtrate was spun dried, and a residue was purified by silica gel chromatography (dichloromethane: methanol=10: 1) to obtain a compound XV (white solids, 34 mg, yield: 76%): ¹H NMR (300 MHz, Pyridine-*d₅*) δ 6.14 (s, 1H), 5.49 (s, 1H), 4.93 (d, *J* = 4.9 Hz, 1H), 4.77 - 4.72 (m, 1H), 4.67 - 4.54 (m, 3H), 4.41 - 4.23 (m, 4H), 3.89 - 3.77 (m, 1H), 3.43 - 3.22 (m, 2H), 1.65 (d, *J* = 6.0 Hz, 3H), 1.32 (s, 3H), 1.20 (s, 3H), 1.08 (s, 3H), 1.03 (s, 3H), 1.01 (s, 3H), 0.97 (s, 3H), 0.86 (s, 3H).

### Embodiment 3

### Synthesis of hederin A2 (XVI, CAS: 3391-80-8)

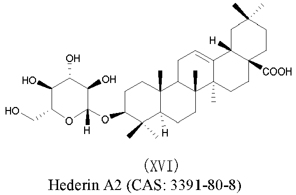

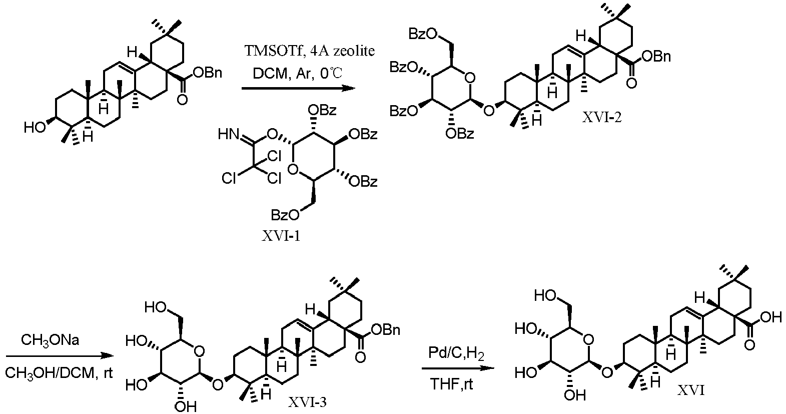

A compound XVI-1 was prepared with reference to methods in literatures (J. Am. Chem. Soc., 1999, 121(51): 12196).

With reference to the methods in Embodiment 1, with benzyl oleanolate as a raw material, XIV-4 was replaced by the compound XVI-1, compounds XVI-2 and XVI-3 were prepared in sequence, and finally, the compound XVI-1 was prepared with catalytic hydrogenation and debenzylation on the compound XVI-3: ¹H-NMR (*pyridine-d*₅) δ 5.44 (br s, 1H), 4.93 (d, 1H, J=7.7), 4.57 (br d, 1H, *J*=11.3)*,* 4.43 (m, 1H), 4.25 (m, 2H), 4.01 (m, 2H), 3.46 (dd, 1H, J=12.5, 2.2), 3.37 (dd, 1H, J=8.0, 2.4), 1.31, 1.30, 1.01, 0.98, 0.93, 0.91, 0.80 (s, 7×3H, CH3); ESI-MS: 641.4 [(M+Na)⁺]. HRMS: m/z 617.4071 [M-H]⁻ ([C₃₆H₅₇O₈] = 617.4059).

### Embodiment 4

Activation activity of pentacyclic triterpenoid saponin on AMPK in Huh-7 cells

The activation activity of a compound on AMPK in Huh-7 cells was detected by using Western Blot.

Cell culture conditions: Huh-7 cells were cultured in an incubator with 5% CO₂ at 37°C in DMEM complete medium (containing 10% fetal calf serum and 1% streptomycin/penicillin).

Antibody: anti-AMPK (CST, 2532S); anti-pAMPK (CST, 2535S).

Western Blot experiment: the influence of the compound on phosphorylation level of the AMPK in the Huh-7 cells was detected: cells with more than 90% cells alive were taken for the experiment. In a 12-well plate, the Huh-7 cells were plated in the plate at 250000 cells per well, the plate was placed in the incubator with 5% CO₂ at 37°C for culture, an original culture medium was discarded after 12 h, a complete medium containing the pentacyclic triterpenoid saponin was added, a final concentration of subject pentacyclic triterpenoid saponin was set to be 10 µM, and an administration time was set to be 12 h. A positive control compound employs AICAR (100 µM), metformin (70 µM) or A-769662 (100 µM). Then proteins were extracted for Western Blot detection.

Experimental result: a Western Blot experimental result was subjected to gray scale scanning, a p-AMPK/AMPK ratio of negative control DMSO was defined as 1, and a pAMPK/AMPK ratio of the subject compound was a relative ratio of a negative control group. The larger the numerical value was, the stronger the activation activity of the AMPK of the compound was. An activity data result is shown in Table 1.

**Table 1 Activation activity of pentacyclic triterpenoid saponins on AMPK (The final concentration of the pentacyclic triterpenoid saponin is set to be 10 µM)**

| Compound | pAMPK/AMPK ratio |
|---|---|
| DMSO | 1.0 |
| Metformin (70 µM) | 1.22 |
| AICAR (100 µM) | 1.47 |
| A-769662 (100 µM) | 2.59 |
| (I) | 2.39 |
| (II) | 2.90 |
| (III) | 1.54 |
| (IV) | 1.75 |
| (V) | 1.81 |
| (VI) | 2.27 |
| (VII) | 1.76 |
| (VIII) | 2.0 |
| (IX) | 1.88 |
| (X) | 3.33 |
| (XI) | 1.95 |
| (XII) | 2.45 |
| (XIII) | 2.51 |
| (XIV) | 2.23 |
| (XV) | 2.87 |
| (XVI) | 2.05 |

The experimental result (Table 1) shows that all the compounds represented by the formulas (I)-(XVI) have very strong AMPK activation activity which are significantly superior to recognized AMPK activators such as the metformin, the AICAR and the A-769662.

### Embodiment 5

Influence of pentacyclic triterpenoid saponins on AMPK downstream signaling pathways of Huh-7 cells

The influence of a compound on AMPK downstream signaling pathways of Huh-7 cells was detected by using Western Blot.

Cell culture conditions: Huh-7 cells: a DMEM complete medium (containing 10% fetal calf serum and 1% streptomycin/penicillin) were cultured in an incubator with 5% CO₂ at 37°C.

Antibody: anti-ACC (CST, 3676S); anti-pACC (CST, 11818S); anti-mTOR (CST, 2983S); anti-pmTOR (CST, 5536S); anti-PGC1a (Abcam, ab54481); anti-ERRa (Abcam, ab76228).

Western Blot experiment: the influence of the compound on the AMPK downstream signaling pathways of the Huh-7 cells was detected.

Cells with more than 90% cells alive were taken for the experiment. In a 12-well plate, the Huh-7 cells were plated in the plate at 250000 cells per well, the plate was placed in the incubator with 5% CO₂ at 37°C for culture, an original culture medium was discarded after 12 h, a complete medium containing the pentacyclic triterpenoid saponin was added, a final concentration of subject pentacyclic triterpenoid saponin was set to be 10 µM, and an administration time was set to be 12 h. A positive control compound employed AICAR (100 µM). Then proteins were extracted for Western Blot detection, and the protein change conditions of pACC/ACC, pmTOR/mTOR, PGC-1a and ERRα were detected.

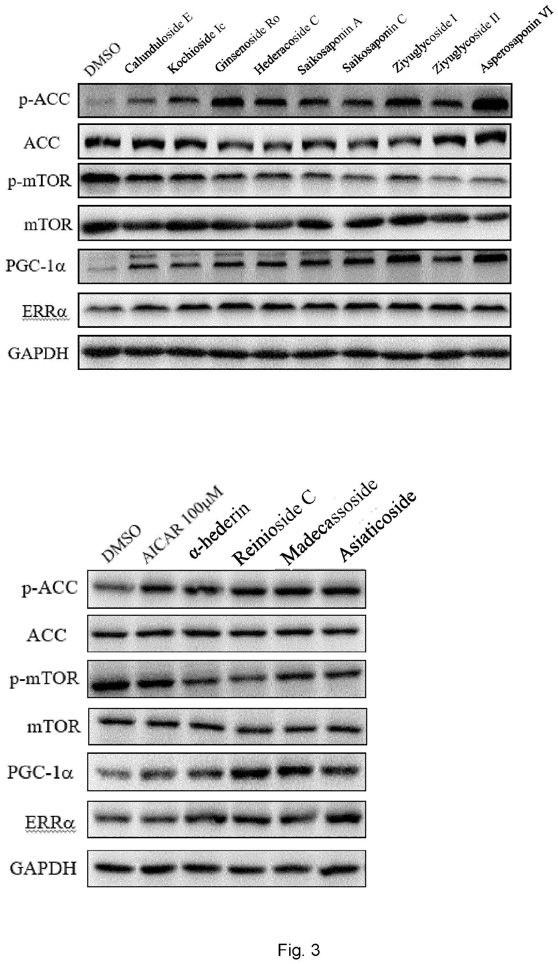

The experimental result (Fig. 3) shows that the pentacyclic triterpenoid saponin compounds of the present invention can effectively cause changes of AMPK downstream proteins, and a change trend is related to AMPK activation. It should be particularly noted that, as being capable of inhibiting mTOR signaling pathways and increasing protein levels of the PGC1α and the ERRα at the same time, the pentacyclic triterpenoid saponin may promote mitochondrial synthesis and oxidative metabolism, induce protective autophagy and then exert the efficacy of reducing blood fat, resisting inflammation, apoptosis and fibrosis and the like.

### Embodiment 6

Protection effect of ginsenoside Ro (compound represented by the fomula (I)) on high fat diet (HFD) induced NASH rat model

Animal: 32 SD male rats of SPF level with 6 weeks old and 220 g were purchased from Beijing Vital River Laboratory Animal Technology Co., Ltd. All the animals maintained 12 h alternative circadian rhythm with free access to food.

Instrument: animal weight scale; slicing machine; full-automatic biochemical analyzer; inverted microscope

Reagent: the ginsenoside Ro (I) was purchased from Shanghai Boka Chemistry Technology Co., Ltd; obeticholic acid (OCA) was purchased from Jiangsu Vcare PharmaTech Co., Ltd; high fat diets were purchased from Research Diets (D12492, 60 kcal%); and standard feeds were purchased from Research Diets (D12450B, 10 kcal%).

Experimental process:
1. Animal grouping and modeling

The rats were randomly divided into 4 groups based on the weight: a control group (CHOW), a high fat diet model group (HFD), a positive drug obeticholic acid (OCA, 5 mg/Kg) group (HFD+OCA) and a ginsenoside Ro (Ro for short, 5 mg/kg) group (HFD+ Ro). The control group was fed with the standard feeds and drank water normally; and the rest groups were fed with the high fat diets, drank 10% aqueous sucrose solution for replacing the water and were modeled for 20 weeks.

### 2. Administration

After 20 weeks, the control group and the high fat diet model group were given with a CMC-Na solution with the volume fraction of 0.5% intragastrically every day, the HFD+OCA group was given with the obeticholic acid (5 mg/Kg) intragastrically every day, and the HFD+ Ro was given with the ginsenoside Ro (5 mg/Kg) intragastrically every day. Administration was conducted for 8 weeks, during which, the control group was given with the standard feeds and drank the water normally, and the rest three groups were given with the high fat diets and drank 10% aqueous sucrose solution. The rats in each group were weighed every day, weights, hairs, feces and activity conditions were carefully observed and recorded.

### 3. Material obtaining

The rats were deprived of food but not water for 12 h in advance; and the next morning, blood was taken from eye sockets, the rats were sacrificed, and livers were harvested. Right liver lobule tissues were fixed with 4% paraformaldehyde for HE stained slicing. A part of liver tissues were divided into 3 parts, which were snap-frozen in liquid nitrogen for subsequent detection of other indexes.

### 4. Determination of biochemical indexes

Whole blood was left to still standing for 2 h at room temperature, centrifugation was conducted for 15 min at 3000 rpm, and a serum was collected. 100 Mg of liver tissues were taken and homogenized with 0.9 mL of precooled normal saline, a homogenate was centrifuged for 15 min at 4°C and 3000 rpm, and a supernatant was taken. Both of the serum and the supernatant were sent to Pathologic Department of Hospital of Integrated Traditional Chinese and Western Medicine in Jiangsu Province for determination of levels of triglyceride (TG), total cholesterol (TC), low density lipoprotein cholesterin (LDL-c) and high density lipoprotein cholesterol (HDL-c) in the serum and levels of the TG and the TC in the supernatant of the homogenate of the liver tissues with the full-automatic biochemical analyzer.

### 5. Liver tissue slicing

Pretreated tissues were sent to Google Biotechnology Co., Ltd for preparing HE stained slices and oil red stained slices.

### 6. Extraction of proteins from liver tissues and Western Blot (WB) detection

Liver tissues preserved at -80°C were put in the liquid nitrogen, about 10 mg of the liver tissues were quickly dissected out, 500 µL of precooled tissue lysate was added, and the tissues were homogenized with a homogenizer and then left to lyse on ice for 30 min; and centrifugation was conducted for 15min at 4°C and 12000 rpm, 170 mL of a supernatant was sucked to a clean EP tube, 10 mL of the supernatant was taken for BCA protein quantification, 40 mL of 5*loading buffer was added in the rest protein supernatant for denaturing by boiling, SDS-PAGE electrophoresis was conducted subsequently, the proteins were transferred onto a PVDF membrane, then incubation and elution of a relevant antibody were conducted, and finally, a chemiluminescence apparatus was employed for imaging analysis.

### 7. Extraction of liver tissue RNA and q-PCR detection

Liver tissues preserved at -80°C were put in the liquid nitrogen, about 10 mg of the liver tissues were quickly dissected out, about 500 µL of a precooled Trizol reagent was added, the tissues were homogenized with the homogenizer and then left to lyse on the ice for 15 min, 100 µL of chloroform was added for vigorous shaking for 15 s, and a product was put on the ice for 10min and then was subjected to centrifugation for 15 min at 4°C and 12000 rpm. An upper aqueous phase is transferred into a clean 1.5 mL EP tube, 200mL of isopropanol was added for RNA precipitation, the EP tube was put on the ice for 10 min and then was subjected to centrifugation for 10min at 4°C and 12000 rpm. The supernate was discarded, precipitates were washed one time with 75% ethyl alcohol, a supernate was discarded after centrifugation, and precipitates of RNA dissolved with the water were treated with 40 mL of DEPC. The concentration of RNA was quantified with Nano, a reverse transcription reagent from Takara company was added according to the specification, and mRNA was reverse transcribed into cDNA with a common PCR instrument. Finally, forward primers and reverse primers of target genes, a q-PCR reagent (SYBR Green) and the cDNA were added in a special 96-well plate for q-PCR, and a q-PCR instrument was used for amplification and quantification. A difference between gene expressions was characterized by selecting a ΔΔCt value, and a GraphPad Prism 7 software was employed for data processing and statistical test.

### 8. Experimental results

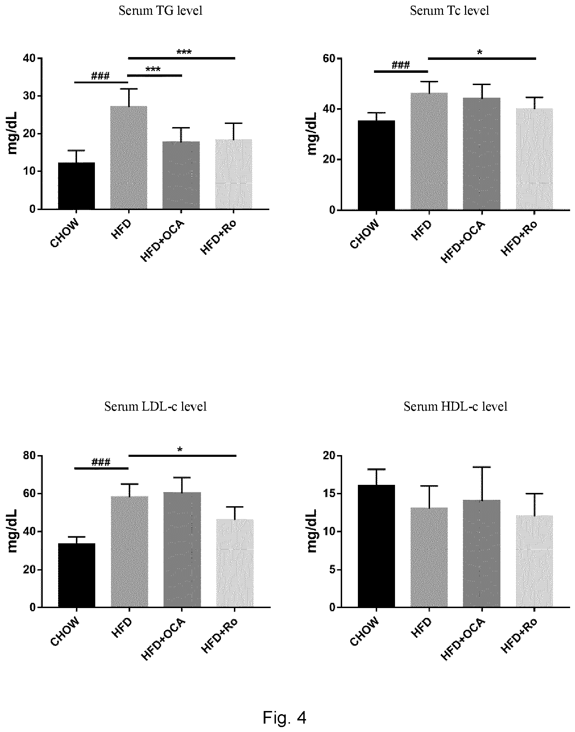

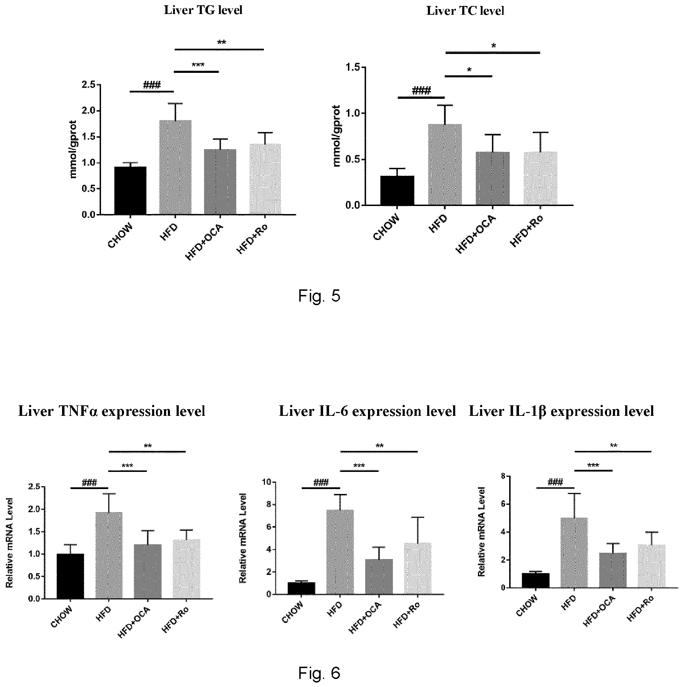

Results in Fig. 4 and Fig. 5 show that OCA and the ginsenoside Ro can both significantly lower the levels of the TG in the serum and the liver, and the curative effect of the ginsenoside Ro is equivalent to that of the OCA. It is noted that the ginsenoside Ro may effectively lower the levels of the TC, the LDL-c and the HDL-c in the serums of the rats fed with the high fat diets, whereas the OCA cannot lower but slightly up-regulates the levels of the TC and the LDL-c in the serums of the rats fed with the high fat diets. In the HFD group, the contents of the TC in the livers were increased to about 3 times of that of the control group and were significantly lowered after the rats were treated with the ginsenoside Ro.

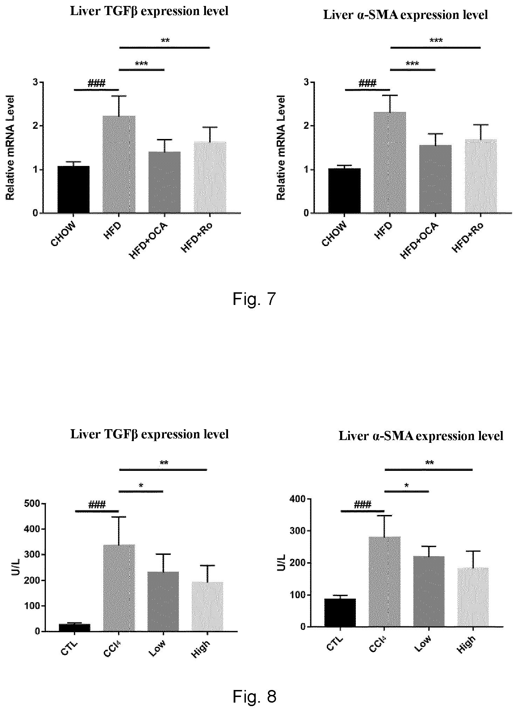

In order to further detect the effect of the ginsenoside RO on lowering inflammations and fibrosis of the livers of NASH animals, the mRNA expression conditions of related inflammatory factors and fibrosis-related cell factors in the liver tissues were determined. A result in Fig. 6 shows that the ginsenoside Ro may significantly inhibit increase, caused by high fat diets, in mRNA expression levels of TNF-α, IL-6 and IL-1β, and therefore, it is considered that the ginsenoside Ro can effectively resist liver inflammatory responses induced by the high fat diets. A result in Fig. 7 shows that the ginsenoside Ro may significantly inhibit increase, caused by the high fat diets, in mRNA expression levels of TGF-β and α-SMA, and therefore, it is considered that the ginsenoside Ro can effectively resist liver fibrosis induced by the high fat diets.

The above results show that the ginsenoside Ro may significantly lower lipid accumulation, caused by the high fat diets, of the rat livers, inhibits the inflammatory responses and the liver fibrosis and has the curative effect equivalent to that of the obeticholic acid (OCA) as a positive control drug. It is noted that, clinical researches and animal experiments discover that the OCA may increase the level of the LDL-c in the serum although lowering lipid accumulation of the livers, whereas the ginsenoside Ro does not have such side effects in the animal experiments. This hints that the ginsenoside Ro may avoid the side effects of the rising the LDL-c in the serum of the OCA in clinical application and thus may be used for preventing and treating chronic hepatic diseases such as fatty liver diseases (particularly, non-alcohol steatohepatitis), chronic hepatitis and hepatic fibrosis.

Other types of the pentacyclic triterpenoid saponin in the present invention also show the fatty liver disease resistant activity similar to the ginsenoside Ro. For example, all of asiaticoside (V), madecassoside (VI), saikosaponin A (VII), saikosaponin D (VIII), ziyuglycoside I (XII) and ziyuglycoside II (XIII) can effectively lower lipid accumulation, caused by the high fat diets, of the rat livers (result shown in Table 2) and inhibit the inflammatory responses of the livers (result shown in Table 3). This hints that the pentacyclic triterpenoid saponin provided by the present invention may be used for preventing and treating the chronic hepatic diseases such as the fatty liver diseases (particularly, non-alcohol steatohepatitis) and chronic hepatitis.

**Table 2 Influence of pentacyclic triterpenoid saponins on lipid levels of serums and livers of high fat diet induced NASH model rats**

| Group | n | Dosage | Liver TG (mmoL/gprot) | Liver TC (mmoL/gprot) | Serum TG (mg/dL) | Serum LDL-c (mg/dL) |
|---|---|---|---|---|---|---|
| Control group | 8 | - | 0.932±0.171 | 0.301±0.081 | 12.52±1.37 | 35.54±3.24 |
| Model group | 8 | - | 1.951±0.319^{###} | 0.881±0.204^{###} | 25.67±3.78^{###} | 57.32±6.22^{###} |
| Ziyuglycoside I | 8 | 5mg/Kg | 1.302±0.443** | 0.547±0.272* | 18.32±2.51** | 48.52±6.98* |
| Ziyuglycoside II | 8 | 5mg/Kg | 1.299±0.387** | 0.503±0.256** | 19.41±4.37* | 45.36±5.37** |
| Saikosaponin A | 8 | 5mg/Kg | 1.268±0.294** | 0.518±0.133** | 20.43±4.04* | 47.06±7.83* |
| Saikosaponin D | 8 | 5mg/Kg | 1.173±0.606*** | 0.509±0.248** | 19.66±5.12* | 46.75±7.35** |
| Madecassoside | 8 | 5mg/Kg | 1.240±0.213** | 0.472±0.201** | 18.83±4.11** | 47.25±7.45* |
| Asiaticoside | 8 | 5mg/Kg | 1.235±0.316** | 0.513±0.279** | 18.00±4.25** | 45.15±4.53** |

| | | | | | | |
|---|---|---|---|---|---|---|
| (n=8, compared with the control group, ^{###}p < 0.001; and compared with the model group, *p < 0.05, **p < 0.01, ***p < 0.001) | | | | | | |

**Table 3 Influence of pentacyclic triterpenoid saponins on levels of ALT (glutamic-pyruvic transaminase), AST (glutamic oxalacetic transaminase) and MDA (malonaldehyde) in serums of high fat diet induced NASH model rats**

| Group | n | Dosage | ALT (U/L) | AST (U/L) | MDA (U/L) |
|---|---|---|---|---|---|
| Control group | 8 | - | 43.94±5.38 | 95.41±8.12 | 7.68±2.47 |
| Model group | 8 | - | 90.36±17.34^{###} | 150.32±24.36^{###} | 16.26±5.18^{###} |
| Ziyuglycoside I | 8 | 5mg/Kg | 74.03±12.38* | 120.91±19.32** | 11.74±3.26* |
| Ziyuglycoside II | 8 | 5mg/Kg | 69.28±12.33** | 122.16±17.38** | 10.41±2.02** |
| Saikosaponin A | 8 | 5mg/Kg | 71.36±9.26* | 115.24±19.23*** | 10.03±3.03** |
| Saikosaponin D | 8 | 5mg/Kg | 67.74±8.29** | 121.43±9.28** | 9.58±3.15** |
| Madecassoside | 8 | 5mg/Kg | 65.86±10.43*** | 127.21±9.34* | 11.59±2.88* |
| Asiaticoside | 8 | 5mg/Kg | 67.38±13.46** | 116.40±11.35*** | 10.14±3.52** |

| | | | | | |
|---|---|---|---|---|---|
| (n=8, compared with the control group, *p < 0.05, **p < 0.01; and compared with the model group, #p < 0.05, ##p < 0.01) | | | | | |

### Embodiment 7

Protection effect of ginsenoside Ro (the compound represented by the formula (I)) on CCl₄-induced hepatic fibrosis mice

Animal: 32 C57BL/6J male mice of SPF level with 25 g were purchased from Beijing Vital River Laboratory Animal Technology Co., Ltd. All the animals maintained 12 h alternative circadian rhythm with free access to food.

Instrument: animal weight scale; slicing machine; full-automatic biochemical analyzer; inverted microscope.

Reagent: the ginsenoside Ro was purchased from Shanghai Boka Chemistry Technology Co., Ltd; CCl₄ was purchased from sigma Company; and sunflower seed oil was purchased from Nanjing Yuanye Biotechnology Co., Ltd.

Experimental process:
1. Animal grouping

The mice were randomly divided into 4 groups based on weights: a blank control group (CTL), a model group (CCI4), a ginsenoside Ro low-dosage group (Low) (5 mg/kg) and a ginsenoside Ro high-dosage group (High) (10 mg/kg), totally 4 groups (with 8 mice each).

### 2. Modeling and administration

A modeling mode was that the mice were given with a CCl₄-contained sunflower seed oil solution (with a CCl₄ content of 25%, v/v) in a dosage of 2mL/kg by intraperitoneal injection, and the mice in the control group were injected with a corresponding dosage of the sunflower seed oil solution two times per week. From experiment day 1 onward, the mice in the control group were given with a CMC-Na solution with the mass fraction of 0.5% intragastrically every day and with sunflower seed oil two times per week by intraperitoneal injection; the mice in the model group were given with the CMC-Na solution with the mass fraction of 0.5% intragastrically every day and with the CCl₄-contained sunflower seed oil solution two times per week by intraperitoneal injection; the mice in the ginsenoside Ro low-dosage group (Low) were given with 5 mg/kg of the ginsenoside Ro intragastrically every day and with a CCl₄ solution two times per week by intraperitoneal injection; and the mice in the ginsenoside Ro high-dosage group (High) were given with 10 mg/kg of the ginsenoside Ro intragastrically every day and with the CCl₄-contained sunflower seed oil solution two times per week by intraperitoneal injection. Administration was conducted for a total of 4 weeks.

### 3. Material obtaining

The mice were deprived of food but not water for 12 h in advance; and the next morning, blood was taken from eyeballs, the mice were sacrificed, and livers were harvested. Right liver lobule tissues were fixed with 4% paraformaldehyde for HE stained slicing. The rest liver tissues were divided into 3 parts, which were snap-frozen in liquid nitrogen for subsequent detection of other indexes.

### 4. Determination of biochemical indexes

Whole blood was left to still standing for 2 h at room temperature, centrifugation was conducted for 15 min at 3000 rpm, and a serum was collected and sent to Pathologic Department of Hospital of Integrated Traditional Chinese and Western Medicine in Jiangsu Province for determination of levels of glutamic oxalacetic transaminase (AST) and glutamic-pyruvic transaminase (ALT) in the serum with a full-automatic biochemical analyzer.

### 5. Liver tissue slicing

Pretreated tissues were sent to Google Biotechnology Co., Ltd for preparing HE stained slices.

### 6. Extraction of liver tissue RNA and q-PCR detection

Liver tissues preserved at -80°C were put in the liquid nitrogen, about 10 mg of the liver tissues were quickly dissected out, about 500 µL of a precooled Trizol reagent was added, the tissues were homogenized with the homogenizer and then left to lyse on the ice for 15min, 100µL of chloroform was added for vigorous shaking for 15 s, and a product was put on the ice for 10 min and then was subjected to centrifugation for 15 min at 4°C and 12000 rpm. An upper aqueous phase was transferred to a clean 1.5 mL EP tube, 200 mL of isopropanol was added for RNA precipitation, the EP tube was put on the ice for 10min and then was subjected to centrifugation for 10 min at 4°C and 12000 rpm. The supernate was discarded, precipitates were washed one time with 75% ethyl alcohol, a supernate was discarded after centrifugation, and precipitates of RNA dissolved with the water were treated with 40 mL of DEPC. The concentration of RNA was quantified with Nano, a reverse transcription reagent from Takara Company was added according to the specification, and mRNA was reverse transcribed into cDNA with a common PCR instrument. Finally, forward primers and reverse primers of target genes, a q-PCR reagent (SYBR Green) and the cDNA were added in a special 96-well plate for q-PCR, and a q-PCR instrument was used for amplification and quantification. A difference between gene expressions was characterized by selecting ΔΔa Ct value, and a GraphPad Prism 7 software was employed for data processing and statistical test.

### 7. Experimental results

As shown in Fig. 8, compared with the blank control group (CTL), the levels of the ALT and the AST in the serums of the model group (CCl₄) were significantly increased; and after the mice were treated with the ginsenoside Ro low-dosage group (Low) (5 mg/kg) and high-dosage group (High) (10 mg/kg), the levels of the ALT and the AST in the serums were both significantly lowered. The above results hint that the ginsenoside Ro may exert certain beneficial effects to the liver inflammations and the hepatic fibrosis by lowering the levels of transaminase ALT and AST in the serums.

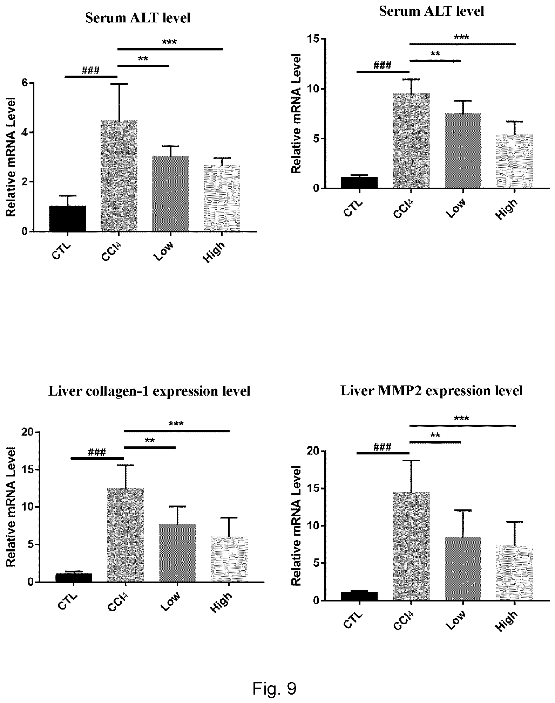

As shown in Fig. 9, compared with the blank control group (CTL), the mRNA expression levels of the hepatic fibrosis related cell factors (TGF □, □-SMA, MMP2 and collagen 1) of the model group (CCl₄) were significantly increased; and after the mice were treated with the ginsenoside Ro low-dosage group (Low) (5mg/kg) and high-dosage group (High) (10 mg/kg), the mRNA expression levels of the hepatic fibrosis related cell factors (TGF□, □-SMA, MMP2 and collagen 1) were significantly lowered. The above results hint that the ginsenoside Ro may inhibit the hepatic fibrosis by lowering the expression levels of the hepatic fibrosis related cell factors (TGF □, □-SMA, MMP2 and collagen 1).

The above results show that the ginsenoside Ro may significantly inhibit the inflammatory responses and the hepatic fibrosis of hepatic fibrosis animal models and improve liver functions. Therefore, considered that the ginsenoside Ro may be used for preventing and treating the hepatic fibrosis and other diseases related to fibrosis.

Other types of pentacyclic triterpenoid saponin in the present invention also show the activity similar to the ginsenoside Ro in the aspect of inhibiting the inflammatory responses and the hepatic fibrosis of the hepatic fibrosis animal models. For example, α-hederin (X), hederacoside C (XI), ziyuglycoside I (XII) and ziyuglycoside II (XIII) can all effectively inhibit mouse hepatic fibrosis induced by CCl₄ (see Table 4). This hints that the pentacyclic triterpenoid saponin provided by the present invention may be used for preventing and treating the hepatic fibrosis and other diseases related to fibrosis.

**Table 4 Influence of pentacyclic triterpenoid saponin on hepatic fibrosis related gene expression of CCl₄-induced hepatic fibrosis mice**

| Group | n | Dosage | TGF-β | collagen 1 | MMP2 |
|---|---|---|---|---|---|
| Control group | 8 | - | 1.00±0.45 | 1.00±0.37 | 1.00±0.27 |
| Model group | 8 | - | 4.42±1.53### | 12.35±3.30### | 14.36±4.39### |
| Ziyuglycoside I | 8 | 5mg/Kg | 2.91±1.10* | 7.65±2.43** | 8.38±3.86* |
| Ziyuglycoside II | 8 | 5mg/Kg | 3.03±0.76* | 8.16±3.37* | 7.38±3.48** |
| α-hederin | 8 | 5mg/Kg | 2.75±0.99** | 7.93±2.28** | 6.37±5.73** |
| Hederacoside C | 8 | 5mg/Kg | 2.96±1.01* | 8.07±3.27* | 7.65±4.03** |

| | | | | | |
|---|---|---|---|---|---|
| (n=8, data in the table is fold induction compared with the control group. Compared with the control group, ###p < 0.01; and compared with the model group, *p < 0.05, **p < 0.01) | | | | | |

### Embodiment 8

### Treatment effect of ziyuglycoside II (compound represented by the formula (XIII)) on ulcerative colitis model mice induced by dextran sulphate sodium salt (DSS)

C57BL/6 male mice with 6-8 weeks old were selected; a control group was fed with purified water; and a model group, a treatment group and a positive control group were fed with purified water containing 2% dextran sulphate sodium salt (DSS) (36000-50000 M.Wt) for 7 days and then with the purified water for 3 days, and drug intervention was conducted at the same time. The mice in the control group and the model group were orally administered with carboxymethyl cellulose sodium as a placebo, the mice in the treatment group were orally administered with the ziyuglycoside II (purchased from Shanghai Boka Chemistry Technology Co., Ltd) (with a dosage of 5 mg/kg), and the mice in the positive control group were orally administered with 5-aminosalicylic acid as a clinical drug (5-ASA, purchased from Shanghai Sangon Biological Engineering Co., Ltd) (with a dosage of 100 mg/kg). In the administration process, weight changes, feces characters and hematochezia conditions of the mice were observed and recorded every day and were scored, and disease activity indexes were calculated (Disease activity index, DAI).

A scoring rule and a calculation method were as follows: DAI=(weight score+diarrhea score+occult blood score)/3, wherein weight score: 0 (weight gained or unchanged), 1 (weight lost by 1-5%), 2 (weight lost by 6-10%), 3 (weight lost by 11-15%), 4 (weight lost by 15% or above); diarrhea score: 0 (normal feces), 2 (soft feces), 4 (feces sticking anuses or diarrhea); and occult blood score: 0 (negative occult blood), 2 (positive occult blood), and 4 (visible bleeding).

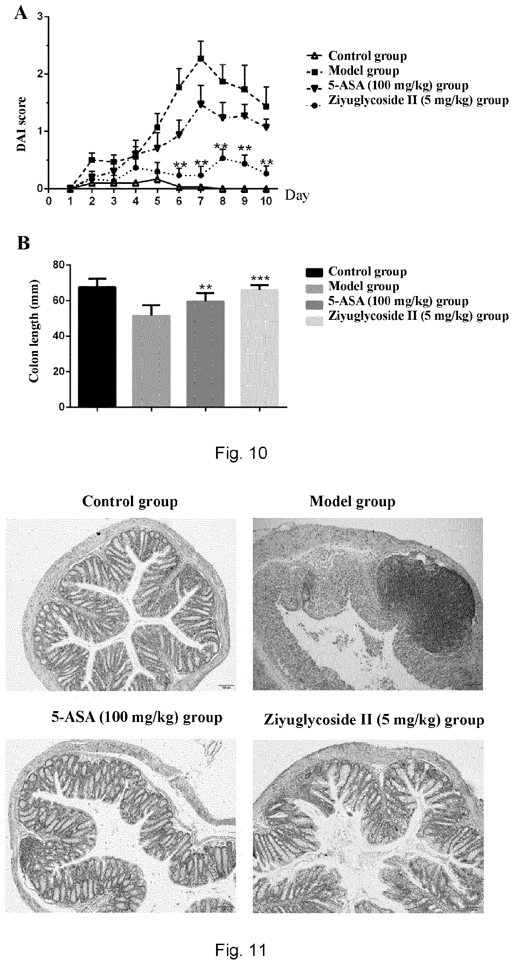

Comparing differences in DAI of various groups, it can be known that the ziyuglycoside II has significant prevention and treatment effects on ulcerative colitis of the mice modeled by the dextran sulphate sodium salt (DSS). As shown in Fig. 10A, after the mice were treated with the ziyuglycoside II (5 mg/kg), DAI scores of the mice were obviously lower than that of the model group , and the curative effect of the ziyuglycoside II treatment group was significantly superior to a positive drug group.

The above mice were sacrificed by cervical dislocation at day 11, colon tissues were harvested, lengths of the colon tissues were measured for statistical summary, and differences in colon length of various groups were compared, from which, the ziyuglycoside II has significant prevention and treatment effect on the ulcerative colitis model mice. As shown in Fig. 10B, the colon lengths of the ziyuglycoside II treatment group were significantly larger than those of the model group and superior to the positive drug group. Surprisingly, there is nearly no difference in colon length of the mice in the ziyuglycoside II treatment group from the control group.

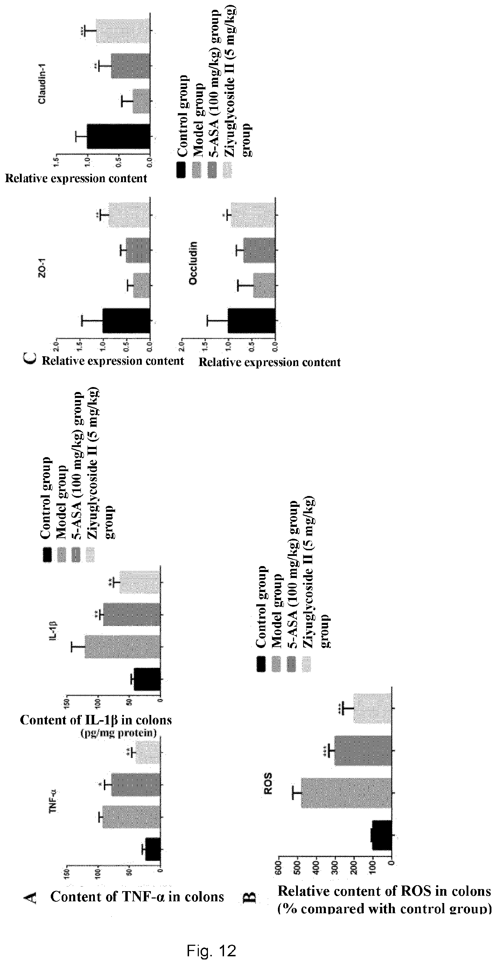

Middle segments, with lengths of about 1cm, of the colon tissues of the above mice were selected, soaked in a formalin solution for fixing overnight, embedded with paraffin and sliced, and the slices were subjected to HE staining. As shown in Fig. 11, patterns of intestinal mucosal layers of the model group are disturbed, and a great amount of inflammatory cells infiltrate into the intestinal mucosal layers; whereas the patterns of the intestinal mucosal layers of the treatment groups are integral, and infiltration of the inflammatory cells is reduced, showing obvious treatment effect. Particularly, compared with the positive drug group, the ziyuglycoside II has the advantage of maintaining a thickness of a muscular layer.
10 mg of proximal rectum segments of the colon tissues of the above mice were selected, PBS was added for homogenization, and centrifugation was conducted for taking a supernate. The supernate was subjected to ELISA detection, and protein quantification was conducted for inflammatory factors TNF-a and IL-1b. As shown in Fig. 12A, the expression levels of the inflammatory factors TNF-□ and IL-1□ of the ziyuglycoside II treatment group were significantly lower than those of the model group, and the effect is superior to the positive drug group.

Under the pathological conditions of the ulcerative colitis, the content of reactive oxygen species (ROS) in colons may be continuously increased, and excessive ROS may strengthen destruction of the colon tissues. 10 mg of colon tissues of the above mice were selected for detection of ROS therein. As shown in Fig. 12B, the content of the ROS in the tissues may be significantly lowered with administration with the ziyuglycoside II (5 mg/kg), and the effect is superior to the positive drug group.

When the ulcerative colitis occurs, the inflammatory factors may destruct tight junction between intestinal tracts, and destruction of the tight junction enables functions of mucosal barriers to to be lost and accelerates development of the diseases. 5 mg of colon tissues of the above mice were selected for qPCR analysis. As shown in Fig. 12C, after the mice were given with the ziyuglycoside II (5 mg/kg), the expression levels of three main proteins, including ZO-1, Claudin-1 and Occludin, related to tight junction are significantly increased compared with the model group. This hints that the ziyuglycoside II further has the efficacy of increasing the expression level of the tight junction and then protecting a structure of each colon.

The above results show that the ziyuglycoside II has significant protection and treatment effect on the ulcerative colitis of the DSS-induced mice, and the total curative effect is significantly superior to the 5-aminosalicylic acid as a clinical first-line drug. Therefore, the ziyuglycoside II is expected to be useful for preparing the drug for preventing or treating the inflammatory bowel diseases such as the ulcerative colitis.

Other types of pentacyclic triterpenoid saponin in the present invention also show the protection effect similar to the ziyuglycoside II on ulcerative colitis models of the DSS-induced mice. For example, after the mice were treated with the saikosaponin A (VII), the saikosaponin D (VIII), ziyuglycoside I (XII), ziyuglycoside II (XIII), ginsenoside Ro (I), asperosaponin VI (II), reinioside C (IX), α-hederin (X) or hederacoside C (XI), DAI scores of the treatment group were all obviously lower than those of the model group and significantly superior to the positive drug group.

### Embodiment 9

### Treatment effect of ziyuglycoside II on 2, 4, 6-trinitrobenzene sulfonic acid (TNBS) modeled Crohn's disease model mice

C57BL/6 male mice with 6-8 weeks old were selected and randomly divided into a control group, a mode group, a treatment group and a positive control group. Expect for the control group, after the mice in the rest groups anesthetized with isoflurane, hoses were carefully inserted into colons of the mice from anuses, and 100 mL of 1.5% 2,4,6-trinitrobenzene sulfonic acid (TNBS) was slowly injected. Before the hoses were pulled out, the mice were placed upside down for 1min. First intragastric administration was conducted at the day of enema treatment, the mice in the control group and the model group were orally administered with carboxymethyl cellulose sodium as a placebo, the mice in the treatment group were orally administered with the ziyuglycoside II (purchased from Shanghai Boka Chemistry Technology Co., Ltd) (with a dosage of 5 mg/kg), and the mice in the positive drug group were orally administered with 5-aminosalicylic acid as a clinical drug (5-ASA, purchased from Shanghai Sangon Biological Engineering Co., Ltd) (with a dosage of 100 mg/kg). Administration was continuously conducted for 7 days. In the administration process, the states of the mice were observed every day, and weight changes of the mice were recorded.

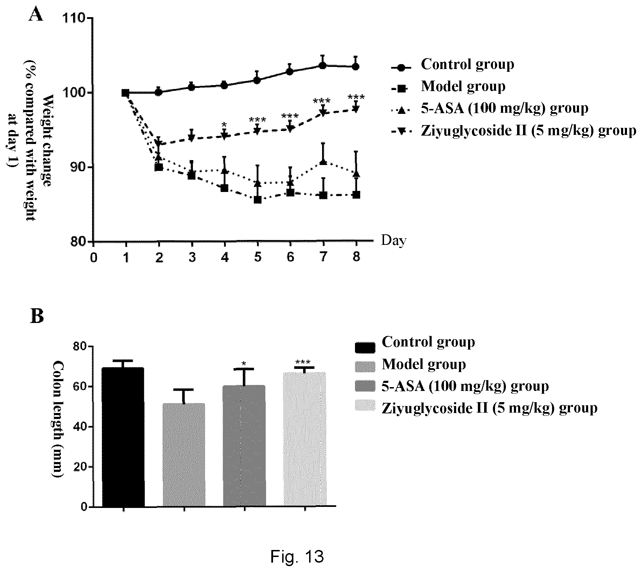

Differences in weight change of the mice in various groups were compared, from which, ziyuglycoside II has the treatment effect on the 2, 4, 6-trinitrobenzene sulfonic acid (TNBS) modeled Crohn's disease model mice. As shown in Fig. 13A, after the mice were given with the ziyuglycoside II (5 mg/kg), the drop scope of weight loss of the mice was obviously lower than that of the model group, and the curative effect of the ziyuglycoside II treatment group was significantly superior to the positive drug group.

The above mice were sacrificed by cervical dislocation at day 8, colon tissues were harvested, lengths of the colon tissues were measured for statistical summary, and differences in colon length of various groups were compared, from which, the ziyuglycoside II has significant treatment effect on the Crohn's disease model mice. As shown in Fig. 13B, the colon lengths of the ziyuglycoside II treatment group were significantly larger than those of the model group and superior to the positive drug group. Surprisingly, there is nearly no difference in colon length of the mice in the ziyuglycoside II treatment group from the control group.

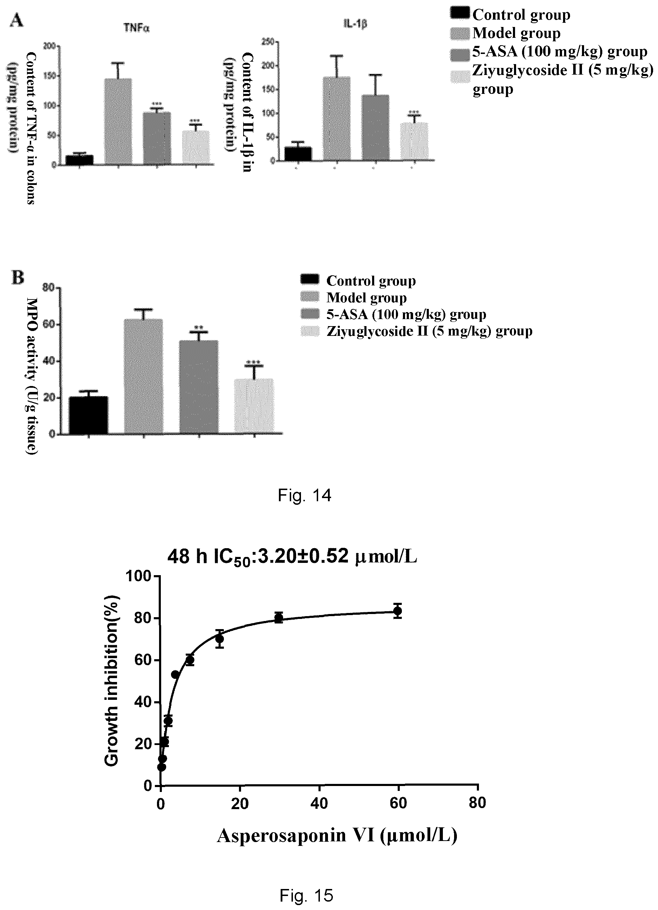

10 mg of proximal rectum segments of the colon tissues of the above mice were selected, PBS was added for homogenization, and centrifugation was conducted for taking a supernate. The supernate was subjected to ELISA detection, and protein quantification was conducted for inflammatory factors TNF-□ and IL-1 □. As shown in Fig. 14A, the expression levels of the inflammatory factors TNF-□ and IL-1□ of the ziyuglycoside II treatment group were significantly lower than those of the model group and superior to the positive drug group.

Myeloperoxidase (MPO) is an enzyme specific to neutrophils, and the amount of the neutrophils in the intestinal tracts may be reflected by detecting the MPO level. 5 mg of proximal rectum segments of the colon tissues of the above mice were selected, PBS was added for homogenization, and the content of the MPO in a homogenate was detected with a MPO kit (purchased from Nanjing Jiancheng Bioengineering Institute). As shown in Fig. 14B, the content of the MPO in the tissues may be significantly lowered with administration with the ziyuglycoside II (5 mg/kg), and the effect is superior to the positive drug group.

The above results show that the ziyuglycoside II has significant protection and treatment effect on the TNBS-induced Crohn's disease model mice, and the total curative effect is significantly superior to the 5-aminosalicylic acid as the clinical first-line drug. Therefore, the ziyuglycoside II is expected to be useful for preparing the drug for preventing or treating the inflammatory bowel diseases such as the Crohn's disease.

Other types of pentacyclic triterpenoid saponin in the present invention also show the protection and treatment effect similar to the ziyuglycoside II on TNBS-induced Crohn's disease model mice. For example, after the mice were treated with the saikosaponin A (VII), the saikosaponin D (VIII), ziyuglycoside I (XII), ziyuglycoside II (XIII), ginsenoside Ro (I), asperosaponin VI (II), reinioside C (IX), α-hederin (X) or hederacoside C (XI), there is nearly no difference in colon length of the mice in the treatment group from the control group. Furthermore, the expression levels of the inflammatory factors TNF-α and IL-1β of the treatment group were significantly lower than those of the model group and superior to the positive drug group.

### Embodiment 10

### Influence of asperosaponin VI (compound represented by the formula (II)) on proliferation of human polycystic kidney cyst lining epithelial cells

Cell and reagent: complete media for human ADPKD cyst lining epithelial cell lines WT9-12 and DMEM were purchased from ATCC Company; fetal calf serums were purchased from Gibco Company; Cell Counting Kit-8 was purchased from DOJINDO Company; Click-iT^{®} EdU Imaging Kits were purchased from InvitrogenTM Thermo Fisher Scientific Company; Caspase -3/CPP32 Colorimetric Assay Kit was purchased from BioVision Company; Annexin V/PI double-staining flow type cell apoptosis detection kits were purchased from Bender Company; and anti-Phospho-(AMPKa, mTOR, p70S6K, 4E-BP1) antibodies were purchased from Cell Signaling Technology Company.

The influence of 2-DG on proliferation of the polycystic kidney epithelial cells was detected with a CCK-8 method: a DMEM complete medium containing 10% fetal calf serum (FBS) was prepared. The polycystic kidney epithelial cells were cultured in an incubator with 5% CO₂ at 37°C after being recovered, a solution was replaced every other day, the polycystic kidney epithelial cells were passaged every 2-3 days, and the cells were in a growing state of adhering to the wall. After trypsin enzyme digestion, cells were resuspended by using a complete medium, the concentration of a polycystic kidney epithelial cell suspension was regulated to be 3×10⁷/L, the polycystic kidney epithelial cell suspension was inoculated to 96-well plate by 100 mL per well for culture for 24 h, and the suspension was replaced by a culture medium containing 0.1% FBS for synchronization for 24 h. The asperosaponin VI was dissolved into DMSO to prepare 20 µM of mother liquor, a drug liquid was diluted by the culture medium, administration concentrations were 0.234 µM, 0.469 µM, 0.938 µM, 1.875 µM, 3.75 µM, 7.5 µM, 15 µM, 30 µM and 60 µM respectively, 6 sub-wells were arranged for each concentration, and then different concentrations of the asperosaponin VI were given to various groups (only the culture medium was added for the blank group without cells and drugs; and the control group was a solvent control, and the DMSO contained culture medium consistent to the administration group was added) for culture for 48 h, 10 mL of Cell Counting Kit-8 reagent was added into each well for incubation for 4h in the incubator at 37°C, and an absorbance value of each well at a wavelength of 450 nm was read by a microplate reader. Proliferation inhibition rate (%)=(A control-A exmpriment)/(A control-A blank)x100%. Experimental results are shown in Fig. 15.

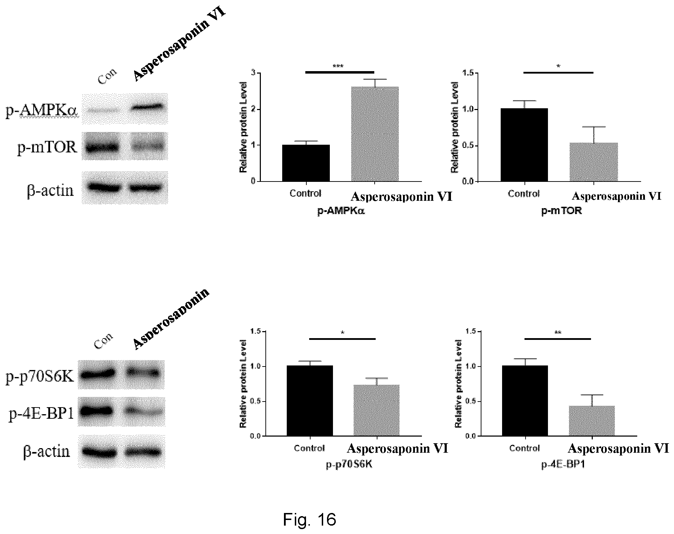

Detection on protein expression with Western blot: cells in the control group and the treatment group (intervening the polycystic kidney epithelial cell with 10 µM of asperosaponin VI for 48 h) were collected, cells were lysed with a RIPA lysate for extraction of total proteins, the concentration of proteins was detected with a BCA method, and 50 mg of sample proteins were taken for sample addition in sequence; after SDS-PAGE electrophoresis, the sample proteins were transferred onto PVDF membranes at a constant current (I=250 mA), were blocked for 1 h with 5% BSA and were incubated for 24 h with different primary antibodies; after the membranes were washed four times with TBST, incubation was conducted for 1.5 h at a normal temperature by using HRP-labeled second antibodies (1: 10000); and after the membranes were washed four times with TBST, the proteins were developed with an ECL, and protein bands of various groups were subjected to gray scale analysis with an Image-J gel image analysis software. Experimental results are shown in Fig. 16.

### Experimental results

CCK-8 experimental results (Fig. 15) show that with increase in concentration of the asperosaponin VI, the number of viable cells is gradually reduced, which hints that the asperosaponin VI is dose-dependent in the inhibition effect on proliferation of the polycystic kidney epithelial cells, and a median inhibitory concentration (IC₅₀) is calculated to be 3.2 µM.

Western blot detection results show that after the mice are treated with the asperosaponin VI, the phosphorylation level of AMPKa in the polycystic kidney epithelial cells is increased (Fig. 16), which shows that the asperosaponin VI activates the AMPK in the polycystic kidney epithelial cells; and meanwhile, the phosphorylation levels of activation molecules mTOR, p70S6K and 4E-BP1 in proliferation-related signaling pathways are lowered (Fig. 16), which shows that the asperosaponin VI inhibits mTOR signaling pathways by activating the AMPK and then inhibits proliferation of the polycystic kidney epithelial cells.

The above results show that the asperosaponin VI may inhibit proliferation of the polycystic kidney epithelial cells and then may prevent and treat the polycystic kidney diseases (particularly, autosomal dominant polycystic kidney disease).

Other types of pentacyclic triterpenoid saponin in the present invention also show the proliferation inhibition effect similar to the asperosaponin VI on the polycystic kidney epithelial cells (Table 5).

**Table 5 Proliferation inhibition effect of pentacyclic triterpenoid saponin on polycystic kidney epithelial cells**

| Group | n | Inhibition rate (%) |
|---|---|---|
| Control group | 6 | 6.8±2.3 |
| Ginsenoside Ro | 6 | 74.4±2.3*** |
| Ziyuglycoside I | 6 | 69.2±1.7*** |
| Ziyuglycoside II | 6 | 72.1±2.0*** |
| Asiaticoside | 6 | 68.5±1.8*** |
| Madecassoside | 6 | 76.3±2.3*** |
| α-hederin | 6 | 80.5±3.2*** |
| Hederacoside C | 6 | 64. 4±2.6*** |

| | | |
|---|---|---|
| (After the polycystic kidney epithelial cells were treated for 48 h with 10 µM pentacyclic triterpenoid saponin, the proliferation inhibition rate of the polycystic kidney epithelial cells was detected with a CCK-8 method; and compared with the control group, ***P < 0.001) | | |

### Embodiment 11

Any one of the compounds (50 g) represented by the formula (I)-(XVI), hydroxypropyl methylcellulose E (150 g), starch (200 g), a proper amount of povidone K30 and magnesium stearate (1 g) were mixed, pelletized and tableted to prepare tablets.

Meanwhile, the mixture could be prepared into oral liquids and capsules based on the oral solution conventional preparation method, the capsule and soft capsule conventional preparation method and other preparation methods in Chinese Pharmacopoeia (2015, 4th edition); or prepared into different dosage forms such as powder, granules, a pill, an injection, a syrup, an inhalant, an ointment, a suppository, a patch and other pharmacally conventional preparation forms by being combined with different carriers including an excipient, an adhesive, a disintegrating agent, a lubricant, a corrigent, a flavoring agent, a coloring agent and a sweetener. Various dosage forms of any one of the compounds represented by the formula (I)-(XVI) may be used for preventing and treating fatty liver diseases, including non-alcoholic fatty liver diseases, particularly, non-alcoholic fatty liver, non-alcoholic steatohepatitis and non-alcoholic steatohepatitis induced hepatic cirrhosis.

Pharmaceutically acceptable salts of any one of the compounds represented by the formula (I)-(XVI), metal ions (such as sodium, potassium and calcium) or salts formed by pharmaceutically acceptable amine (such as ethylendiamine and tromethamine) or ammonium ions may also be used for preventing and treating the fatty liver diseases, including the non-alcoholic fatty liver diseases, particularly, the non-alcoholic fatty liver, the non-alcoholic steatohepatitis and the non-alcoholic steatohepatitis induced hepatic cirrhosis.

Any one of the compounds represented by the formula (I)-(XVI) or pharmaceutically acceptable salts or solvates thereof may be used in combination with one or more drugs for resisting the non-alcoholic fatty liver diseases to be used for preventing and treating the fatty liver diseases, including the non-alcoholic fatty liver diseases, particularly, the non-alcoholic fatty liver, the non-alcoholic steatohepatitis and the non-alcoholic steatohepatitis induced hepatic cirrhosis with significant effect. These drugs include AMPK activators, farnesoid X receptor (FXR) activators (such as obeticholic acid, GS-9674, EDP-305 and LJN452), acetyl-CoA carboxylase (ACC) inhibitors (such as GS-0976), apoptosis signal regulating kinase-1 (ASK1) inhibitors (such as Selonsertib), PPAR activators (such as Elafibranor, Saroglitazar, IVA337 and MSDC-0602K), caspase inhibitors (such as Emricasan), stearoyl-CoA desaturase-1 (SCD1) inhibitors (such as Aramchol), long-acting glucagon-like peptide-1 (GLP-1) receptor activators (such as Semaglutide), apical sodium-dependent bile salt transporter (ASBT) inhibitors (such as Volixibat), vascular adhesion protein-1 (VAP-1) inhibitors (such as BI 1467335), CCR5R blocking agents (such as Cenicriviroc) and thyroid hormone receptor b (THR-b) activators (such as MGL-3196).

## Claims

1. A use of pentacyclic triterpenoid saponins represented by formulas (I)-(XVI) or pharmaceutically acceptable salts or solvates thereof in preparing a drug for preventing or treating AMPK and/or ERRa-mediated diseases:

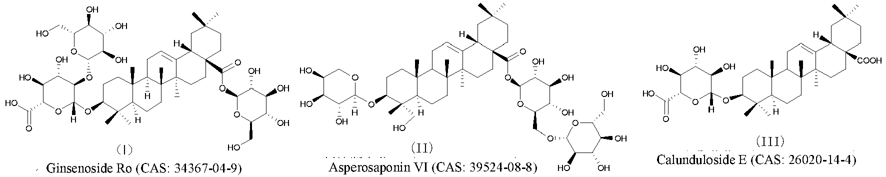

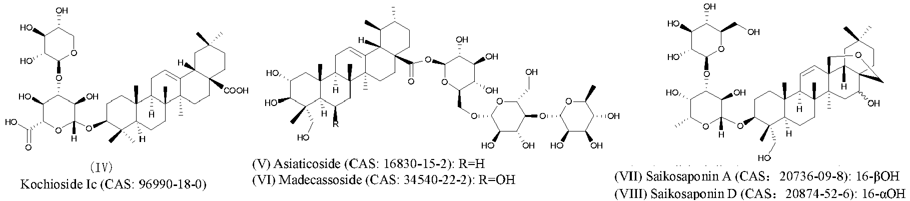

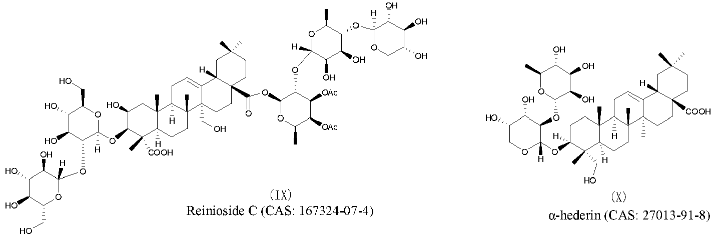

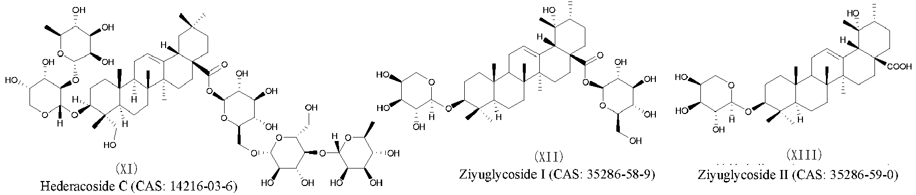

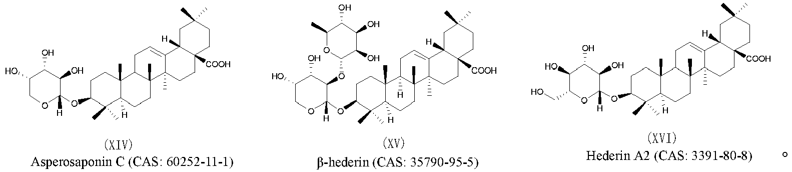

2. The use according to claim 1, wherein the use of the pentacyclic triterpenoid saponins represented by the formulas (I)-(XIII) or the pharmaceutically acceptable salts or solvates thereof in preparing the drug for preventing or treating the AMPK-mediated diseases is:

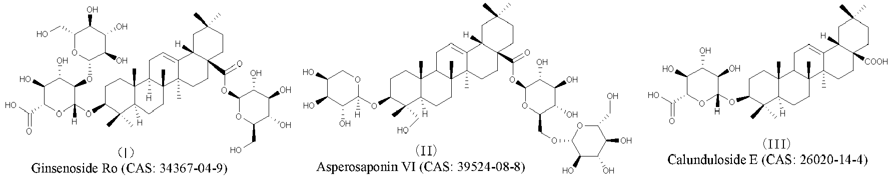

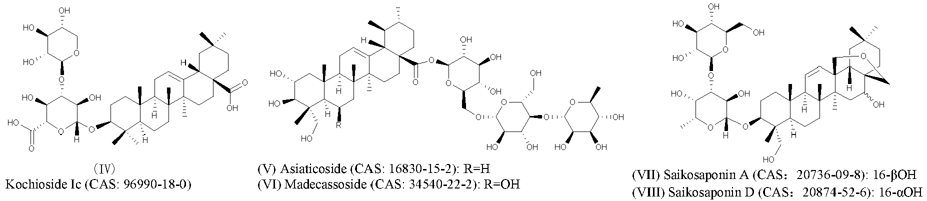

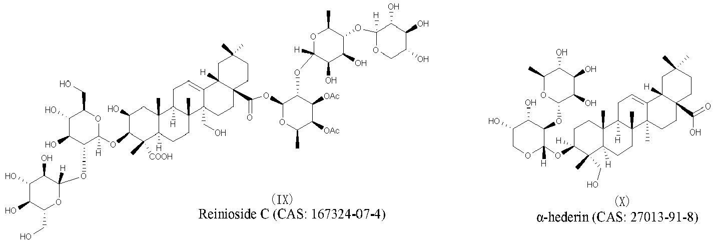

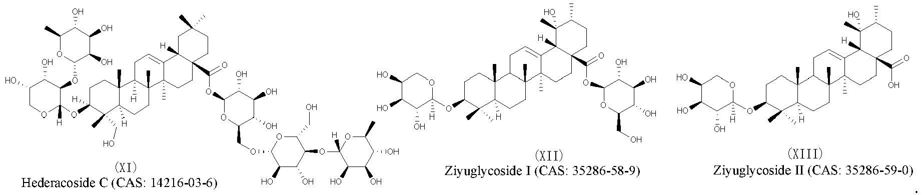

3. The use according to claim 1 or 2, wherein the AMPK and/or ERRa-mediated diseases comprise hepatic diseases, respiratory system diseases, metabolic disorders, autoimmune diseases, cardiovascular and cerebrovascular diseases, kidney diseases, central nervous system diseases or muscular dystrophy.

4. The use according to claim 3, wherein the hepatic diseases comprise non-alcoholic fatty liver, non-alcoholic steatohepatitis, alcoholic fatty liver disease, hepatic fibrosis, hepatic cirrhosis, primary biliary cholangitis or primary sclerosing cholangitis.

5. The use according to claim 3, wherein the respiratory system diseases comprise cough, asthma, chronic obstructive pulmonary disease, bronchitis, pneumonia, respiratory distress syndrome, emphysema, idiopathic pulmonary fibrosis, cystic fibrosis pulmonary disease, allergic rhinitis or chronic rhinitis.

6. The use according to claim 3, wherein the metabolic disorders comprise insulin resistance, metabolic syndromes, diabetes and complications thereof, hyperlipidemia, obesity, hyperuricemia, gout or osteoporosis.

7. The use according to claim 3, wherein the autoimmune diseases comprise ulcerative colitis, Crohn's disease, systemic lupus erythematosus, rheumatoid arthritis, psoriasis, multiple sclerosis or Behcet's disease.

8. The use according to claim 3, wherein the cardiovascular and cerebrovascular diseases comprise hypertension, atherosclerosis, arrhythmia, coronary heart diseases, myocardial infarction, myocardial hypertrophy, carditis, heart failure, pulmonary arterial hypertension, cerebral apoplexy, stroke, cerebral infarction or cerebral edema.

9. The use according to claim 3, wherein the kidney diseases comprise autosomal recessive polycystic kidney disease or chronic nephritis.

10. The use according to claim 3, wherein the central nervous system diseases comprise Parkinson diseases, Alzheimer's diseases, α-synucleinopathy, depression, amyotrophic lateral sclerosis, fibromyalgia syndrome, neuralgia, Down's syndrome, Hallervorden-Spatz diseases, Huntington's diseases or Wilson's disease.

11. The use according to claim 3, wherein the muscular dystrophy is Duchenne muscular dystrophy.

12. A drug composition for preventing or treating the AMPK and/or ERRa-mediated diseases, comprising one or more compounds represented by formulas (I)-(XVI) or pharmaceutically acceptable salts or solvates thereof as active ingredients and pharmaceutically acceptable adjuvants.

13. The drug composition according to claim 12, wherein a dosage form of the drug composition is a capsule, powder, a tablet, a granule, a pill, an injection, a syrup, oral liquid, an inhalant, a cream, an ointment, a suppository or a patch.
